(19) Europäisches Patentamt
European Patent Office
Office européen des brevets

(11) **EP 2 204 218 B1**

(12) **EUROPÄISCHE PATENTSCHRIFT**

(45) Veröffentlichungstag und Bekanntmachung des Hinweises auf die Patenterteilung:
**25.01.2012  Patentblatt 2012/04**

(51) Int Cl.:
***A61N 5/06*** (2006.01)

(21) Anmeldenummer: **10003440.4**

(22) Anmeldetag: **27.05.2004**

(54) **Anordnung zur Reduktion von Mikroorganismen**

Assembly for reducing microorganisms

Agencement de réduction de microorganismes

(84) Benannte Vertragsstaaten:
**AT BE BG CH CY CZ DE DK EE ES FI FR GB GR HU IE IT LI LU MC NL PL PT RO SE SI SK TR**

(30) Priorität: **28.05.2003  DE 10324644
23.10.2003  DE 10349710**

(43) Veröffentlichungstag der Anmeldung:
**07.07.2010  Patentblatt 2010/27**

(62) Dokumentnummer(n) der früheren Anmeldung(en) nach Art. 76 EPÜ:
**04739394.7 / 1 626 777**

(73) Patentinhaber: **bredent medical GmbH & Co. KG
89250 Senden (DE)**

(72) Erfinder:
• **Vizethum, Freimut Dr.
D-69231 Rauenberg (DE)**
• **Schütze, Reinhold
A-4800 Attnang-Puchheim (AT)**

(74) Vertreter: **Schmitt, Meinrad
Reble, Klose & Schmitt GbR
Patent- und Rechtsanwälte
Postfach 12 15 19
68066 Mannheim (DE)**

(56) Entgegenhaltungen:
DE-A1- 3 918 965    DE-A1- 10 049 999
JP-A- 11 155 638    US-A- 4 785 805
US-A- 5 029 581    US-A- 5 454 794

EP 2 204 218 B1

**Beschreibung**

Die Erfindung bezieht sich auf eine Anordnung zur Reduktion von Mikroorganismen gemäß der im Oberbegriff des Patentanspruchs 1 angegebenen Merkmale.

Aus der DE 100 49 999 A1 ist eine derartige Anordnung bekannt, welche als Apparat zur photodynamischen Therapie und Diagnostik ausgebildet ist. Das Bestrahlungsgerät ist als Tischgerät ausgebildet, mit welchem mittels einer Steckverbindung ein Lichtleiter verbindbar ist. Das Bestrahlungsgerät enthält einen als Buchse ausgebildeten ersten Verbindungskörper. Der von einem Schutzmantel umgebene Lichtleiter bildet einen Applikator mit einem zweiten Verbindungskörper der lösbaren Steckverbindung. Mit dem distalen Ende des Lichtleiters ist über eine weitere Steckverbindung ein Vorsatz mit Blendschutz verbindbar, mittels welchem eine Blendung des Patienten oder des Bedienungspersonals vermieden werden soll. Der Vorsatz ist in einer gewünschten Position über dem zu therapierenden Bereich fest zu positionieren, wobei mittels einer Kühlvorrichtung in dem genannten Bereich die Temperatur zur Vermeidung von Schmerz und Gewebeschädigungen niedrig gehalten wird. Zur Ausbildung des Lichtleiterendes bzw. der Lichtaustrittsfläche sind keinerlei Angaben entnehmbar. Als Lichtquelle ist eine 150 W Projektorleuchte vorgesehen, wobei durch dichriotische Filter das Licht auf eine schmale Bande zwischen 610 nm und 740 nm eingeschränkt ist.

Aus der US 5 029 581 A ist ein Apparat für die Lasertherapie bekannt, welcher in einem Steuergerät einen rohrförmigen Körper aufweist und ferner eine Anzahl von Lichtleiterkabeln aufweist. Die Lichtleiterkabel sind durch das Steuergerät hindurchgeführt, wobei die proximalen Enden der Lichtleiter im rohrförmigen Körper angeordnet sind, welcher ferner eine Laserdiode enthält. An den distalen Enden des jeweiligen Lichtleiterkabels ist jeweils eine zusätzliche Sonde angeordnet. Weder am Steuergerät noch an dem rohrförmigen Körper sind Verbindungskörper vorgesehen. Ggf. können außerhalb des Steuergeräts die Lichtleiterkabel Verbindungskörper enthalten, um den zu therapierenden Bereichen, beispielsweise den Händen oder Füßen, eines Patienten die jeweils gewünschte Sonde zuordnen zu können.

In der DE 39 18 965 A1 ist ein Laserbehandlungsgerät beschrieben, welches zum Führen eines Laserstrahls zum Schneiden, Verdampfen oder Koagulieren eines bestimmten Gewebes oder zur photochemischen Behandlung ausgebildet ist. Von einer Laserstrahlquelle werden Laserstrahlen über einen Lichtleiterteil zu einem spitzen Teil geführt, aus welchem das Licht austritt. Ein nahes Ende des Lichtleiters kann mittels eines optischen Verbinders mit der Laserstrahlquelle verbunden werden. Das Spitzenteil ist mit dem distalen Ende des Lichtleiters einstückig verschweißt. Das Spitzenteil besteht aus einem lichtübertragenden Material mit niedriger thermischer Leitfähigkeit und hohen Wärmewiderstandseigenschaften, um Verunreinigungen und Einbrennverluste infolge der Berührung des Spitzenteils mit dem zu behandelnden Gewebe zu vermeiden.

In der US 5 454 794 A ist ein steuerbarer lichtverteilender Katheder bekannt, dessen Lichtleiter mit einer Lichtquelle lösbar verbindbar ist. Das distale Ende des Lichtleiters endet in einer Spitze, welche optisch diffundierendes Material enthält, wobei der umgebende Körper des Katheders eine abgerundete Spitze aufweist. Ein von einer Umhüllung freies Ende oder Spitze des Lichtleiters ist nicht vorhanden.

Ein aus zwei Komponenten bestehendes Lasersystem für chirurgische oder endoskopische Anwendungen ist aus der US 4 785 805 A bekannt. Die eine Komponente enthält ein zylindrisches Rohr mit einem inneren Hohlraum, in welchem eine Linse angeordnet ist und in welchen Laserlicht in den Bereich einer Öffnung gelenkt und fokussiert wird. Die andere Komponente ist lösbar mit der ersten Komponente verbunden und enthält einen Lichtleiter, welcher in die genannte Öffnung der ersten Komponente hineinragt.

Weiterhin sind aus der WO 01/87416 A1 eine Anordnung und ein Verfahren zur Reduktion bzw. Zerstörung von Mikroorganismen, wie Bakterien, unter Einsatz einer lichtaktivierbaren Substanz, nach der photodynamischen Therapie (PDT) bekannt. Mit Hilfe der lichtaktivierbaren Substanz, insbesondere eines Farbstoffes, werden die Mikroorganismen, sensibilisiert und/oder angefärbt, und nach Bestrahlung mit Licht geeigneter Wellenlänge und Energiedichte abgetötet. Das Wirkungsprinzip der PDT beruht, nach der selektiven Einwirkung und/oder Anfärbung der Mikroorganismen auf der physikalischen Wirkung der Energieübertragung auf die lichtaktivierbare Substanz, welche auch als Photosensitizer oder Photosensibilisator bezeichnet wird. Von dort kann die Energie für Reaktionen an der Zellmembran zur Verfügung gestellt werden. Die mittels eines Bestrahlungsgeräts, insbesondere eines Lasergeräts, erzeugte Energie wird damit auf die Mikroorganismen konzentriert und die Gleichgewichtslage von Reaktionen, welche auch im nicht belichteten Zustand im "normalen" Milieu ablaufen, werden verschoben und in der Folge werden die Mikroorganismen zerstört.

Ferner ist aus der EP 0 637 976 B1 die Verwendung einer lichtsensibilisierenden Substanz oder Verbindung bzw. eines Photosensitizers oder Photosensibilisators (PS) bei der Herstellung eines Medikamentes zur Verwendung bei der Desinfizierung oder Sterilisierung von Geweben der Mundhöhle oder einer Wunde oder Läsion in der Mundhöhle durch Zerstören von mit einer Erkrankung verbundenen Mikroben in einer periodontalen Tasche, in der Region zwischen dem Zahn und dem Zahnfleisch bekannt. Hierbei erfolgt eine Kontaktierung der Gewebe, der Wunde oder der Läsion mit dem Photosensitizer, wobei die mit einer Erkrankung verbundenen Mikroben den Photosensitizer aufnehmen. Es wird eine Bestrahlung der Gewebe, der Wunde oder der Läsion mit Laserlicht bei einer durch den Photosensitizer absorbierenden Wellenlänge durchgeführt. Die Keimreduktion dieser kombinierten Farbstoff- und Laserbehandlung wird für verschiedene Keime und Photosensitizer in Form von Lösungen mit unter anderem Methylenblau und Toluidinblau in unterschiedlichen, recht geringen Konzentrationen beschrieben, und zwar von 0,01 bis 0,00125 % (Gewicht pro Volumen), wobei ferner der Einfluß der applizierten Energiedichte aufgezeigt wird. Als Lichtquellen werden HeNe-Laser mit einer Wellenlänge von 634 nm und einer Leistung von 7,3 mW sowie GaAs-Laser mit einer Wellenlänge von 660 nm und einer Leistung von 11 mW verwendet.

Hiervon ausgehend liegt der Erfindung die Aufgabe zugrunde, die Anordnung dahingehend auszubilden, dass eine effektive und steuerbare Therapie mit einem geringen apparativen Aufwand und bei einfacher Handhabung erreicht wird. Die Therapie lokaler, oberflächlicher Infektionen im Mund-, Kiefer- und Gesichtsbereich soll mit einem geringen Aufwand und hoher Funktionalität erfolgen können. Des Weiteren soll eine möglichst homogene Bestrahlung des zu therapierenden Bereiches, insbesondere der Mundschleimhautoberfläche, erreicht werden. Im Hinblick auf die große Verbreitung und der großen Häufigkeit von Infektionen, gerade im Mund-, Kiefer- und Gesichtsbereich einschließlich dentogener Infektionen, sollen die bisher insoweit bestehenden Probleme vermieden oder zumindest verringert werden.

Die Lösung dieser Aufgabe erfolgt gemäß der im Patentanspruch 1 angegebenen Merkmale.

Die erfindungsgemäße Anordnung ermöglicht bei einfachem Aufbau und einfacher Handhabung eine funktionssichere und praxisgerechte Anwendung der Therapie mittels einer lichtaktivierbaren Substanz und einem Bestrahlungsgerät. Die lichtaktivierbare Substanz wird in Lösung in hoher Konzentration bereitgestellt, zweckmäßig abgefüllt in einer Spritze sterilisiert und gebrauchsfertig. Vorteilhaft ist die Konzentration des Photosensitizers in einem Lösungsmittel wie wässrige Lösung oder Alkohol oder Ethanol, mit einem hohen Wert vorgegeben. Die Konzentration, und zwar Gewicht pro Volumen, ist in vorteilhafter Weise größer als 0,1%, zweckmäßig größer als 0,5%, vorgegeben, wobei als Obergrenze vorteilhaft 10%, zweckmäßig 5%, insbesondere 3% vorgegeben ist. Insbesondere hat sich eine Konzentration von zumindest näherungsweise 1% als besonders geeignet erwiesen. Das Bestrahlungsgerät, welches insbesondere als Lasergerät ausgebildet ist, ist kombiniert mit einem Applikationssystem, wobei Applikatoren mit dem Bestrahlungsgerät bevorzugt lösbar verbindbar sind. Die Applikatoren sind in vorteilhafter Weise Einmaloptiken, mittels welchen eine gezielte und exakte Bestrahlung des zu therapierenden Bereiches ermöglicht wird. Die Applikatoren werden nur einmalig zur Behandlung benetzt, so dass insbesondere den Hygieneanforderungen entsprochen wird und eine unerwünschte Übertragung von Mikroorganismen sicher vermieden wird, ohne aufwendige Maßnahmen zur etwaigen nachträglichen oder wiederholten Sterilisierung. Die Applikatoren enthalten Lichtleiter, insbesondere Faserlichtleiter, und ermöglichen problemlos intraoral die Lichtverteilung bzw. Bestrahlung und können als Pocketsonden oder Flächensonden ausgebildet sein. In bevorzugter Weise sind das Bestahlungsgerät und der wenigstens eine Applikator dahingehend ausgebildet, dass eine direkte Einkopplung des Lichtes der Lichtquelle in den Lichtleiter erfolgt. In einer besonderen Ausgestaltung der Erfindung wird ein Lichtleiter bzw. eine Lichtleiterfaser mit hoher numerischer Apertur verwendet, wobei die numerische Apertur größer als 0,5, insbesondere größer als 0,7 ist. Hierdurch entstehen bei der Einkopplung des Lichtes in den Applikator bzw. die Lichtleiter geringe Verluste und gleichzeitig wird gewährleistet, dass der aus dem Applikator bzw. dem Lichtleiter austretende Lichtstrahl stark öffnet.

In einer besonderen Ausgestaltung der Erfindung ist mit dem Bestrahlungsgerät eine Sperreinrichtung derart kombiniert, dass nur bei angekoppeltem Applikator und/oder Lichtleiter aus dem Bestrahlungsgerät Licht austreten kann. Solange der Applikator nicht ordnungsgemäß mit dem Bestrahlungsgerät verbunden ist, wird mittels der Sperreinrichtung der direkte Lichtaustritt aus dem Bestrahlungsgerät unterbunden. In einer besonderen Ausgestaltung enthält das Bestrahlungsgerät, insbesondere dessen Kopfteil, eine bevorzugt zentrale Bohrung, in welche das Lichtleiterende des Applikators eingeführt und fixiert wird. Die Sperreinrichtung ist insbesondere im Strahlengang des Lichtes der Lichtquelle und dem freien Ende und/oder der freien Stirnfläche des Lichtleiterendes angeordnet. Erfindungsgemäß wird die Sperreinrichtung beim Ankoppeln des Applikators und/oder beim Einführen des Lichtleiterendes in die genannte Bohrung derart, insbesondere mittels des Lichtleiterendes, betätigt, dass der Strahlengang freigegeben ist. Die genannte Bohrung und/oder das eingeschobene Lichtleiterende sind bezüglich der Lichtquelle derart angeordnet und/oder ausgerichtet, dass das Licht der Lichtquelle auf die freie Stirnfläche des Lichtleiterendes fällt, gegebenenfalls fokussiert mittels eines optischen Systems. Die Applikatoren enthalten eine Verbindungs- bzw. Steckvorrichtung, insbesondere in Form eines Luersteckers, zur Aufnahme an einem Kopf oder Kopfteil des Bestrahlungsgerätes. Ferner sind die Applikatoren in vorteilhafter Weise zumindest teilweise gebogen derart ausgebildet, dass eine gezielte Bestrahlung der zu therapierenden Bereiche in der Mundhöhle ermöglicht wird. Ferner sind die Applikatoren bevorzugt zumindest teilweise flexibel ausgebildet, so dass unerwünschte Verletzungen vermieden werden.

In einer bevorzugten Ausgestaltung weist der Lichtleiter eine definierte Geometrie des Lichtaustrittsbereiches derart auf, dass der Lichtaustritt an die Form der zu belichtenden Areale des zu therapierenden Bereiches angepasst ist, wobei entweder ein flächiger oder körperhafter, dreidimensionaler Abstrahlungsbereich erzeugt wird. Ferner weist der Applikator und/oder der Lichtleiter an der Spitze einen Abstandshalter auf, mit welchem ein aktiver Lichtkreis des austretenden Lichtes angezeigt und/oder der richtige bzw. vorgegebene Belichtungsabstand festgelegt wird. Gemäß einer weiteren Ausgestaltung weist der Lichtleiter eine derartige Geometrie auf, dass das Eindringen in enge, komplex geformte Körperhöhlen und/oder Gewebstaschen ermöglicht wird und/oder diese sanft geöffnet werden können. Vorteilhaft weist der Lichtleiter eine konisch geformte Spitze auf, und zwar zweckmäßig mit einem Winkel von 1,5 bis 4˚

zur Senkrechten. Darüber hinaus hat es sich als besonders vorteilhaft erwiesen, den Lichtleiter im Bereich der Spitze mit einer Lichtaustrittsfläche vorgegebener Mikrostruktur im Bereich von $10\mu m$ bis $200\mu m$ zu versehen. In bevorzugter Weise weist die Spitze des Lichtleiters eine Mikrorauhigkeit mit einem Ra-Wert im Bereich von 10 bis $40\mu m$, vorzugsweise 20 bis 30 $\mu m$ auf. Des Weiteren ist der Verbindungskörper des Applikators als ein Steck- und/oder Schraubverschluß mit einem integrierten Tiefenanschlag ausgebildet, wodurch eine definierte Positionierung des in das Bestrahlungsgerät eingeführten Lichtleiterendes bezüglich der Lichtquelle in axialer Richtung gewährleistet wird.

Für die erfindungsgemäße Verwendung der Anordnung wird die lichtaktivierbare Substanz, welche bevorzugt Farbstoff enthält, zunächst in hoher Konzentration auf den zu therapierenden Bereich aufgebracht und nachfolgend wird eine Spülung mit einem Medium, insbesondere Wasser und/oder mit einem möglichst basischen pH-Wert durchgeführt. Nachfolgend wird die Bestrahlung mittels des Lichts des Bestrahlungsgeräts durchgeführt, wobei in bevorzugter Weise eine optimierte Zellschädigung erfolgt. Es hat sich als besonders effektiv erwiesen, die lichtaktivierbare Substanz zunächst in hoher Konzentration auf den zu therapierenden Bereich aufzubringen und nachfolgend eine Spülung mit einem Medium, insbesondere Wasser, und/oder mit einem möglichst hohem Sauerstoffpartialdruck durchzuführen und schließlich die Bestrahlung mittels des Lichts der genannten Lichtquelle durchzuführen, wobei bevorzugt eine optimierte Zellschädigung erfolgt. Des Weiteren hat es sich als besonders zweckmäßig erwiesen, dass nach dem Aufbringen der lichtaktivierbaren Substanz in hoher Konzentration auf den zu therapierenden Bereich und ferner vor der Belichtung mittels des Lichts der Lichtquelle eine Verringerung der Menge der lichtaktiven Substanz durchgeführt wird, und zwar insbesondere durch Abwischen und/oder Abtupfen und/oder Absaugen und/oder Abblasen.

Weiterbildungen und besondere Ausgestaltungen der Erfindung sind in den Unteransprüchen angegeben. Nachfolgend wird der Aufbau der Anordnung und deren erfindungsgemäße Verwendung im einzelnen erläutert. Die Anordnung enthält:

1. Lichtaktivierbare Substanz:

Die in Lösung vorliegende lichtaktivierbare Substanz, zum Beispiel Methylenblau, welche bevorzugt einen Farbstoff enthält und als Photosensitizer oder Photosensibilisator bezeichnet wird, ist bevorzugt in eine Spritze abgefüllt und ist sterilisiert und gebrauchsfertig. Zum Aufbringen auf den zu therapierenden Bereich ist insbesondere eine Kanüle 26g vorgesehen, welche insbesondere einen Aussendurchmesser von 0,45mm, eine Länge von 25 bis 40 mm aufweist und insbesondere um 30 bis 45 Grad abgewinkelt und elastisch ausgebildet ist.

2. Bestrahlungsgerät mit Lichtquelle, insbesondere Lasergerät oder Therapielaser, bevorzugt in folgenden Ausführungsformen:

a. Mit optischem System, insbesondere Linsenpaket, und bevorzugt aufschraubbarem Ansatz zur Lichtleiterankopplung.

b. Mit direkter Strahleinkopplung ohne Linsenpaket, mit Lichtraum vor der Diode, so dass ohne angekoppelten Lichtleiter nur wenig Licht diffus aus dem zur Kopplung des Lichtleiters vorgesehenen Zugang fällt. Die Anordnung ist ferner bevorzugt derart, dass bei Verwendung einer Diode mit monitoring das rückgestrahlte Licht dazu führt, dass die Diode abgeregelt wird.

Das Bestrahlungsgerät enthält bevorzugt eine Sperreinrichtung, mittels welcher ein Lichtaustritt unterbunden wird so lange mit dem Bestrahlungsgerät ein Applikator nicht verbunden ist.

3. Applikationssystem

Einmaloptiken oder Applikatoren, welche insbesondere jeweils nur einmal zum Einsatz gelangen und bevorzugt Lichtleiter und Kunststoffummantelung aufweisen. Diese sind bevorzugt flexibel und/oder sterilisiert und/oder gebrauchsfertig und/oder für beide vorstehend genannte Bestrahlungsgeräte kompatibel. Als Material des oder der Lichtleiter ist Glas oder Kunststoff vorgesehen mit großer numerischer Apertur von über 0,5, um möglichst viel Licht einzukoppeln und außerdem das Licht in einem großen Bereich abzustrahlen. Es ist von besonderer Bedeutung, dass aufgrund der lösbaren Verbindung zwischen dem Bestrahlungsgerät und den Applikatoren, der jeweilige Applikator im Rahmen der Erfindung als vergleichsweise einfach aufgebaute und kostengünstige Komponente und als "Wegwerfprodukt" nur einmal zur Verwendung gelangt und danach entsorgt wird.

Zweckmäßig sind zwei Ausführungsformen der Applikatoren vorgesehen:

a. Pocketsonde mit konischem Abstrahlungsbereich insbesondere zum Belichten der Parodontaltasche. In einem Schritt wird die Tasche geöffnet, Gewebe beiseite geschoben und der Bereich belichtet mit Abstrahlung nach vorne und zirkulär. Die Oberfläche ist aufgerauht, um die Abstrahlung des Lichtes diffus zu erreichen (beispielsweise mit Sandpapier 100 geschliffen).

b. Flächensonde zur Belichtung oberflächlicher Areale bevorzugt mit Abstandshalter,

i. dessen Länge den richtigen Abstand zum Gewebe markiert,

ii. dessen Winkel den Bereich markiert, in dem die therapeutisch notwendigen Lichtleistungen aufgebracht werden, wobei die überlappende Bestrahlung eines Bereiches erleichtert wird.

4. Ferner in einer besonderen Weiterbildung einen TherapieController und/oder ein Programm für den PC und/oder eine eigenständige Anzeige und Kontrolleinheit. Diese dienen zur Steuerung und Orientierung des Behandlers während der Therapie. Hiermit wird vor allem die Wahl der Größe der zu belichtenden Fläche oder der Anzahl der Zähne ermöglicht und insbesondere anzeigt:

a. Die Zeit der Einwirkung des Photosensitizers,

b. die Zeit für die Spülung des Areals,

c. die Zeit für die Belichtung je cm2 oder Zahn mit akustischem Hinweis auf das Ende der Belichtung je Zahn oder cm2,

d. das Ende der Behandlung.

Die Komponenten der Anordnung werden nachfolgend erläutert:

Die Energiequelle bzw. Lichtquelle des Bestrahlungsgerätes ist derart ausgebildet, dass im relevanten Wellenlängebereich eine ausreichend hohe Penetration des Lichtes im Gewebe stattfindet, da langwellige Strahlung tiefer ins Gewebe eindringt als kurzwellige. Im Bereich des Absorptionsmaximum der lichtaktivierbaren Substanz, beispielsweise von Methylenbau (664 nm in NaCl oder 655 nm in 96% Ethanol), findet eine ausreichend tiefe Durchdringung des Gewebes mit Licht statt. Im sogenannten optischen Fenster zwischen 600-900 nm wird das Licht sehr gering von Chromophoren wie Hämoglobin oder Melanin absorbiert.

Als Energie- bzw. Lichtquellen sind vor allem Lasersysteme geeignet. Der Laser (LightAmplifikation by Stimulated Emission of Radiation) ist eine Lichtquelle, die monochromatisches, kohärentes und kollimiertes Licht mit hoher Leistung abgeben kann. Unter kohärentem Licht versteht man den sowohl zeitlich als auch räumlich phasengleich verlaufende Wellenzüge.

Im Bereich der erfindungsgemäß vorgegebenen niedrigen Leistungs- bzw. Energiedichten (0,1-100 mW/cm2) sind im Wesentlichen photochemische Prozesse wirksam. Hierbei führt die Absorption von Licht primär nicht zu einer Erwärmung des Gewebes. Diese durch athermische Laserapplikation erzeugten Effekte in biologischen Materialen werden als "laserinduzierte Biostimulation" bezeichnet. Unter Einsatz des Photosensitizers, welcher nachfolgend auch als Photosensibilisator bezeichnet wird, werden derartige Laser als Lichtquelle für die Photodynamische Therapie (PDT) benutzt. Auch bei diesen Lasern können mit höherer Leistungs- bzw. Energiedichte photothermisch induzierte Effekte auftreten.

Bei Dioden-Lasern werden Halbleiterkristalle als aktives Medium verwendet, die bei Anregung kohärente Strahlung im VIS- oder IR-Bereich emittieren. Bei diesen Lasern werden direkt durch elektrischen Strom Photonen erzeugt.

Im Zusammenhang mit dem bevorzugt zum Einsatz gelangenden Photosensitizer wird als Bestrahlungsgerät ein spezieller Diodenlaser eingesetzt, der nachfolgend als HELBO TheraLite bezeichnet wird. Der Diodenlaser HELBO TheraLite ist insbesondere für Methylen Blau geeignet.

Dieses Lasersystem ist durch die folgende Eigenschaften gekennzeichnet:

- Lichtquelle            Diode
- Wellenlänge         660 nm (+5)
- Leistung            max. 100 mW
- Mode            Cw bzw. kontinuierlich
- Lichtausgangsleistung         >40mW<50 mW
- Kühlungssystem            Luft
- Energieversorgung         Batterie oder Akku

Die Übertragung der Licht- bzw. Laserstrahlung wird erfindungsgemäß durch das Applikationssystem ermöglicht. Das Applikationssystem sorgt für die gewünschte Strahlgeometrie am Applikationsort und ermöglicht nicht zuletzt die einfache Handhabung der Laserstrahlung zur Therapie. Teil des Applikationssystems ist die Lichtleitfaser.

Unter der Zielsetzung einer effektiven und steuerbaren Therapie in der Mundhöhle ist eine möglichst homogene Bestrahlung der Mundschleimhautoberfläche erreicht. Die Mundhöhle zeichnet sich jedoch durch eine komplexe Geometrie und durch die Anwesenheit sehr unterschiedlich absorbierender Strukturen wie Knochen, Zähne und Schleimhaut aus, die deutlich von einer planen Geometrie abweichen. Dies wird mit den erfindungsgemäßen Applikatoren gewährleistet.

Lichtleitfasersysteme können die benötigte Energie auch in Areale mit komplexem Zugang wie in der Mundhöhle leiten. Zur Einkopplung in eine Lichtleitfaser wird der Primärstrahl des Lasers im Bestrahlungsgerät entweder direkt oder über ein Linsenpaket auf das Faserende fokussiert. Die numerische Apertur der Faser bestimmt den Einkoppelwinkel derart, dass die Strahlung weitgehend in den Lichtleiter eintritt.

Die Abstrahldivergenz der Lichtleitfaser ist durch die Art der Einkopplung in die Faser Kopf und des Bestrahlungsgerätes ebenfalls durch die numerische Apertur (Sinus des Öffnungswinkels) der Faser selbst bestimmt. Eine höhere Divergenz ermöglicht einen breiteren Abstrahlwinkel.

Falls Fasern mit einem starkem Abfall der Energie bzw. bei stark schwankender Lichtverteilung über die bestrahlten Fläche verwendet werden, wird das Belichtungsfeld häufig überlappend bestrahlt werden.

Im Vergleich zur Bare fiber verfügt die bevorzugt vorgesehene Microlensfaser über ein weitestgehend homogenes Bestrahlungsprofil. Mit einer optimierten Microlensfaser lässt sich eine Leistungsverteilung über die gesamte bestrahlte Fläche mit einer Homogenität von etwa 96 % erreichen. Das Überlappen der Strahlungsfelder ist bei Verwendung einer Microlensfaser nicht erforderlich, sodass die Möglichkeit gegeben ist höchst effizient den infizierten Bereich abzudecken und unnötige Überlappungsbereiche nicht gegeben sind.

DerApplikationsmodus (extraoral oder intraoral) hängt von der Fokusgröße ab, der entsprechend der Ausdehnung des Befundes gewählt wird.

Eine bevorzugte Alternative stellen erfindungsgemäß Fasern mit einer sehr hohen numerischen Apertur dar, mit denen ebenfalls gleichmäßig ausgeleuchtete Areale erzeugt werden können.

Dabei ist erfindungsgemäß eine numerische Apertur von mindestens 0,5, vorzugsweise 0,7 oder höher vorgegeben, um aufwändiges Schleifen der Faserspitzen zu vermeiden und für die Bestrahlung von intraoralen Bereichen einen klinisch sinnvollen Abstand von 0,5 bis 1 cm einhalten zu können.

Im Rahmen der Erfindung wird insbesondere folgendes Applikationssystem mit den Applikatoren angewendet, welche als Pocketsonde und/oder Flächensonde ausgebildet sind. Diese Applikatoren enthalten Kunststofflichtleiter, die in einer Ummantelung eingebettet sind, eine Einkoppelfläche mit Anschluss an das Bestrahlungsgerät, insbesondere Lasergerät, und einen spezifisch geschliffenen und/oder eine Mikrostruktur aufweisenden Abstrahlbereich besitzen.

Die Erfindung wird nachfolgend anhand der besonderen in der Zeichnung dargestellten Ausführungsbeispiele näher erläutert, ohne dass insoweit eine Beschränkung erfolgt.

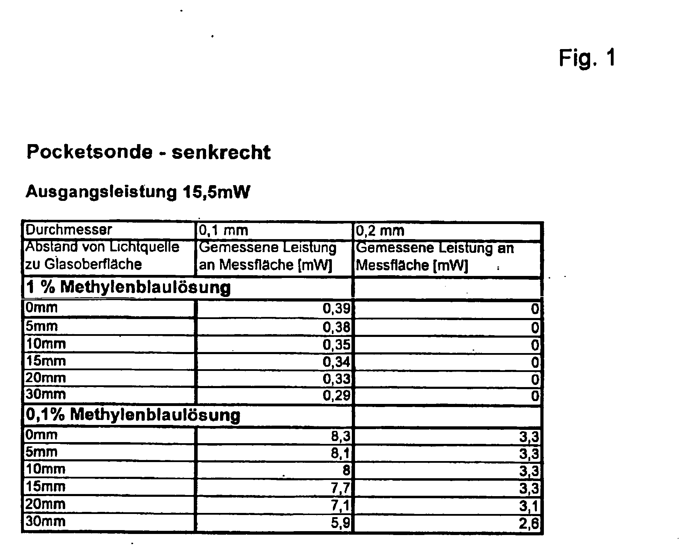

Fig. 1 enthält eine Tabelle von Messergebnissen einer Pocketsonde, welche senkrecht zu einer Glasoberfläche angeordnet war bei einer Ausgangsleistung von 15,5 mW. Hierbei sind in Abhängigkeit des Durchmessers einer Lichtleitfaser unter Berücksichtigung des Abstandes von der Lichtquelle die gemessenen Leistungen in mW angegeben. Es sei hier festgehalten, dass in bevorzugter Weise ein Photosensitizer mit hoher Konzentration, und zwar bevorzugt größer als 0,1%, insbesondere in der Größenordnung von 1% in einem Lösungsmittel zur Verwendung gelangt, wobei gemäß Fig. 1 als Photosensitizer Methylenblau in Lösung vorgesehen ist.

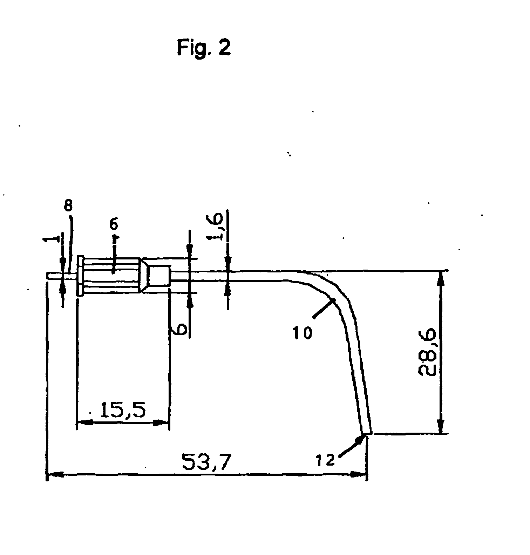

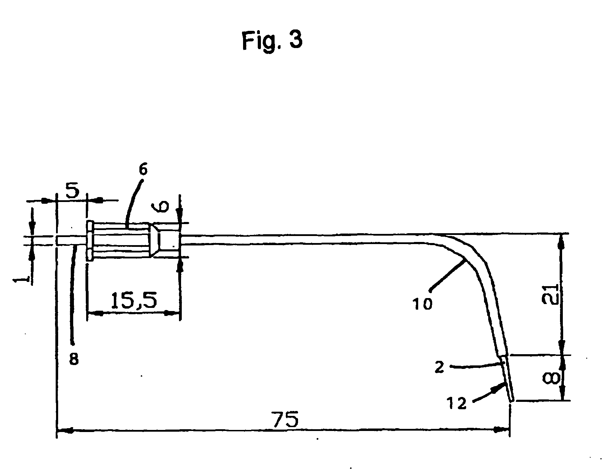

In Fig. 2 bzw. 3 sind als Applikatoren eine Pocketsonde und Flächensonde dargestellt. Es versteht sich, dass die hier ebenso wie in den weiteren Figuren beispielshaft angegebenen Maße in Millimeter bedarfsweise abgeändert werden können. Die Applikatoren enthalten einen als Faser ausgebildeten Lichtleiter 2, welcher größtenteils außen von einem Schutzmantel 4 umgeben ist. Die Applikatoren weisen ferner einen Verbindungskörper 6 auf, welcher bevorzugt als Luerstecker ausgebildet ist und über welchen die Verbindung bzw. Aufnahme im Kopf des Bestrahlungsgerätes erfolgt. Der Lichtleiter 2 ist durch den Verbindungskörper 6 hindurchgeführt und steht über diesen erfindungsgemäß mit seinem Ende 8 mit einer vorgegebenen Länge hinaus, und zwar gemäß Fig. 2 um 5 mm. Das Lichtleiterende 8 ist nicht mit einem Schutzmantel versehen und wird beim Ankoppeln des Applikators in den Kopfteil des Bestrahlungsgeräts eingeführt und in diesem positioniert. Der Lichtleiter 2 ist zumindest teilweise flexibel ausgebildet und/oder enthält einen gebogenen Bereich 10. Im übrigen sind die Abmessungen der Applikatoren derart vorgegeben, dass diese problemlos in die Mundhöhle eingeführt werden können.

Die Laserstrahlung tritt bei der in beiden Applikatoren verwendeten Laser mit 1 mm Kemdurchmesser aufgrund der Materialeigenschaften und derAnschliffgeometrie im Bereich der Spitze 12 unter einem Divergenzwinkel von im Bereich von 30 bis 60˚, bevorzugt 40 bis 55 Grad, nach frontal oder insbesondere bei der Pocketsonde in einem Bereich unter von 220 bis 300 Grad, bevorzugt 240 bis 290˚, insbesondere 260 bis 280 Grad zirkulär aus.

Diese Eigenschaften machen die Applikatoren zu einfach anwendbaren optischen Werkzeugen für die präzise Bestrahlung von einfachen, aber auch von komplex geformten Oberflächen.

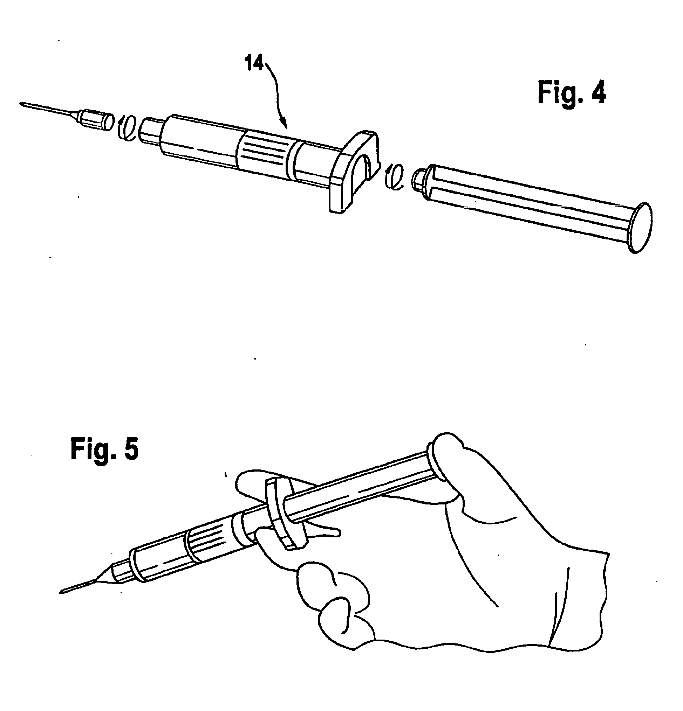

Der Aufbau und die Montage der Applikationsspritze 14, welche die lichtaktivierbare Substanz in Lösung enthält, sind in Fig. 4 und 5 dargestellt. Die die lichtaktivierbare Substanz in Lösung enthaltende Spritze 14 wird gebrauchsfertig geliefert. Vor der Anwendung muß die Verschlusskappe durch vorsichtiges Drehen von der Spitze entfernt und die beigepackte sterile Kanüle auf dem Luer Lock Adapter der Spritze fixiert werden. Auf die richtige Fingerposition ist zu achten. Der Patient ist vor der Behandlung sorgfältig aufzuklären. Nach Durchführung der aus klinischchirurgischen Aspekten gebotenen Maßnahmen zur Vorbereitung des zu therapierenden Bereiches wird die Umverpackung geöffnet, ein Blister mit der lichtaktivierbaren Substanz, ein Blister mit einer Kanüle und gegebenenfalls der Beipackzettel entnommen. Dieser ist vor der Erstanwendung zu lesen. Unter Berücksichtigung steriler Kautelen sind beide Blister über dem sterilen OP-Tray zu entleeren. Spritze und Kanüle sind damit steril und zur Anwendung im sterilen Bereich geeignet. Der Silikonstopfen wird vorsichtig von der Spritze entnommen und die Kanüle durch Eindrehen auf dem Luerkonus fixiert.

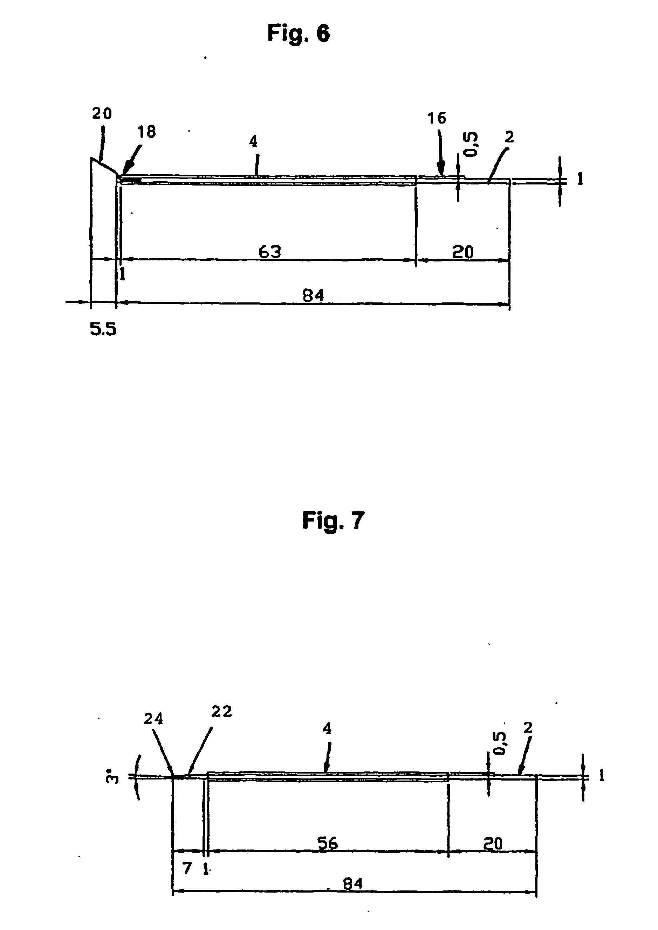

Fig. 6 zeigt das noch nicht gebogene Lichtleiterstück des Applikators ohne den Verbindungskörper, welcher in dem Bereich 16 angeordnet wird. Zwischen diesem Bereich 16 und dem freien Ende 18 ist der Schutzmantel 4 vorgesehen. Am freien Ende 18 ist ferner ein Abstandshalter 20 angeordnet, mittels welchem ein definierter Abstand, hier 5,5 mm, zu dem zu therapierenden Bereich vorgegeben wird.

Fig. 7 zeigt das Lichtleiterstück für die Pocketsonde, wobei das freie Ende 22 frei vorm Schutzmantel 4 ist und eine Länge von 7 mm aufweist. Das freie Ende 22 ist angeschliffen und weist eine Oberfläche entsprechend einer Bearbeitung mit Schleifpapier mit einer Korngröße 100 auf. Die Spitze 24 des freien Endes 22 ist stumpf ausgebildet und/oder mit einem Radius versehen. Die Oberfläche weist in bevorzugter Weise eine vorgegebene Mikrorauhigkeit auf. Diese besitzt vorzugsweise einen Ra-Wert im Bereich zwischen 10 bis 40$\mu$m, vorzugsweise im Bereich zwischen 20 bis 30$\mu$m.

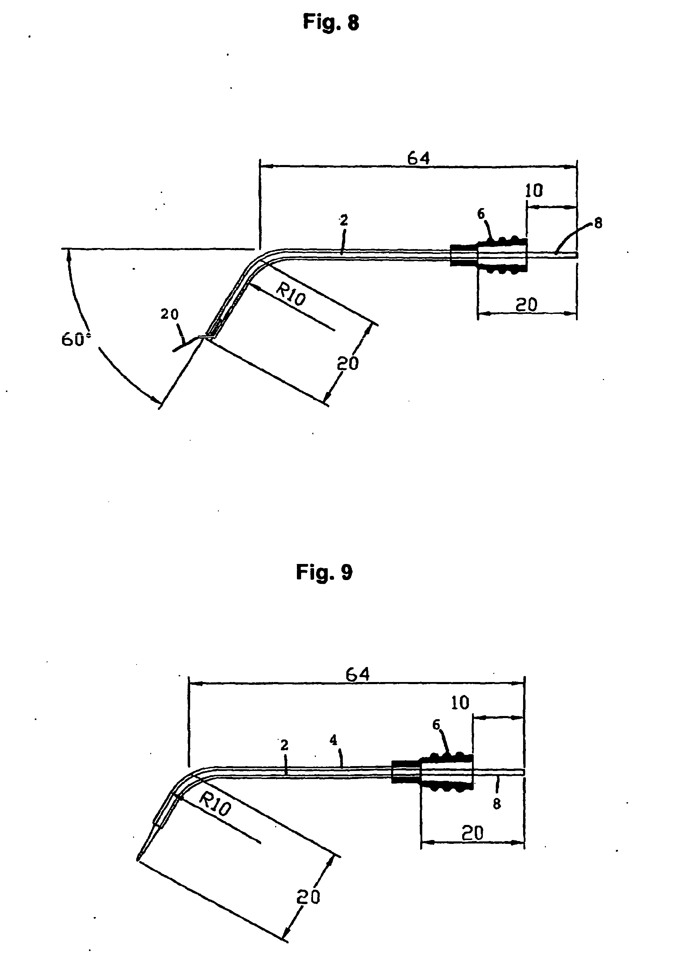

Fig. 8 zeigt einen Applikator ähnlich Fig. 4, wobei am freien Ende ein Abstandshalter 20 angeordnet ist. Das Lichtleiterende 8 ragt bei dieser Ausführungsform aus dem Verbindungskörper 6 heraus, und zwar hier in einer Länge von 10 mm.

In Fig. 9 ist ein gebogenes Lichtleiterstück einer Pocketsonde ähnlich Fig. 3 dargestellt, wobei das freie Lichtleiterende 8 aus dem Verbindungskörper 6 mit einer vorgegebenen Länge, hier 10 mm, herausragt.

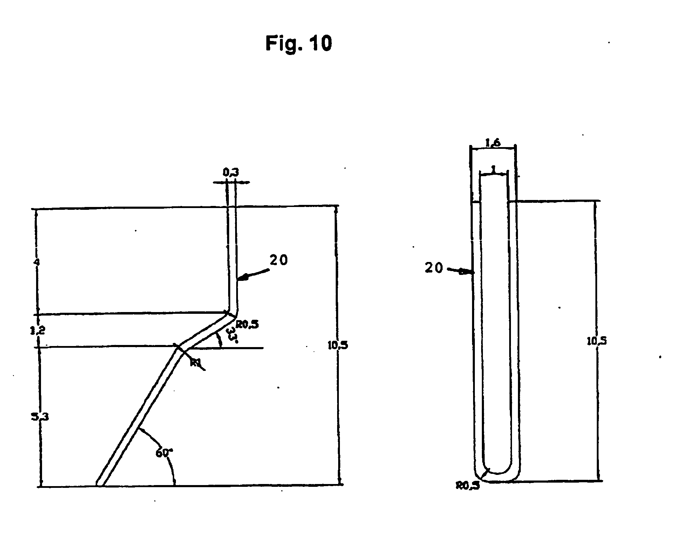

In Fig. 10 sind seitliche Ansichten einer bevorzugten Ausführungsform des Abstandshalters 20 dargestellt.

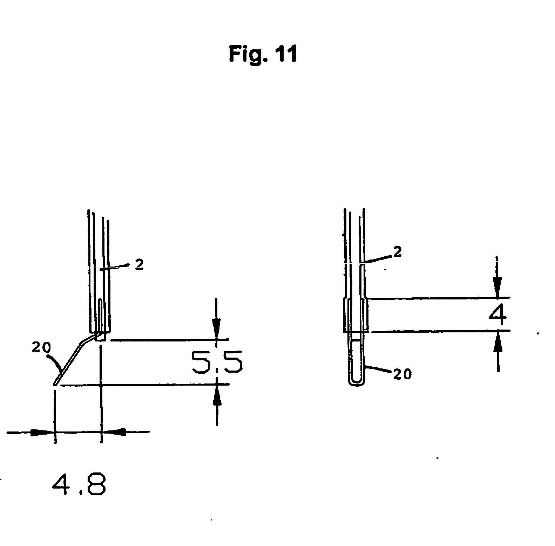

Fig. 11 zeigt teilweise in seitlichen Ansichten die Flächensonde mit eingeschrumpftem Lichtleiter mit dem Abstandshalter 20.

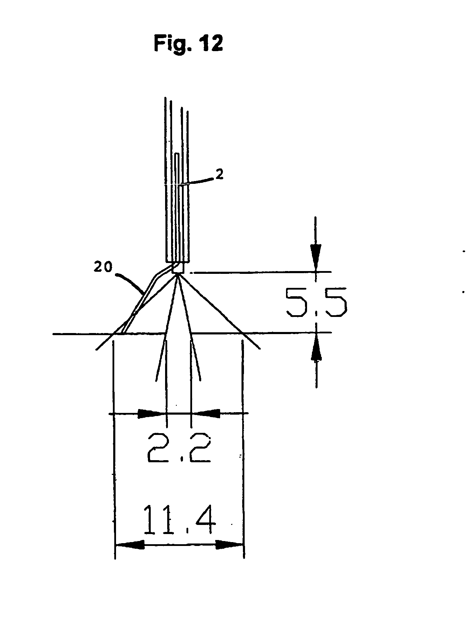

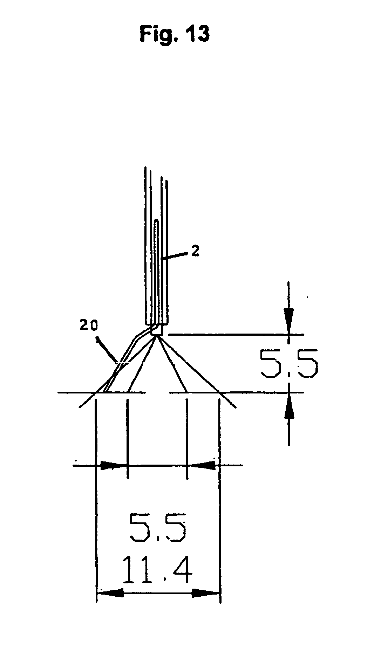

Fig. 12 und 13 zeigen teilweise Flächensonden, wobei gemäß Fig. 12 die Flächensonde eine numerische Apertur von 0,72 aufweist.

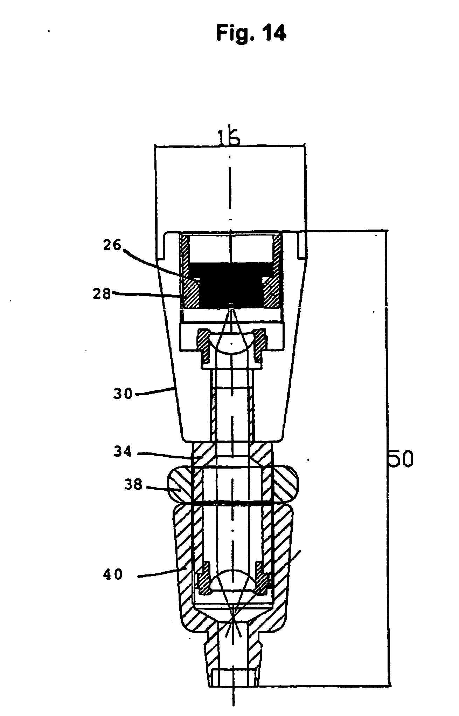

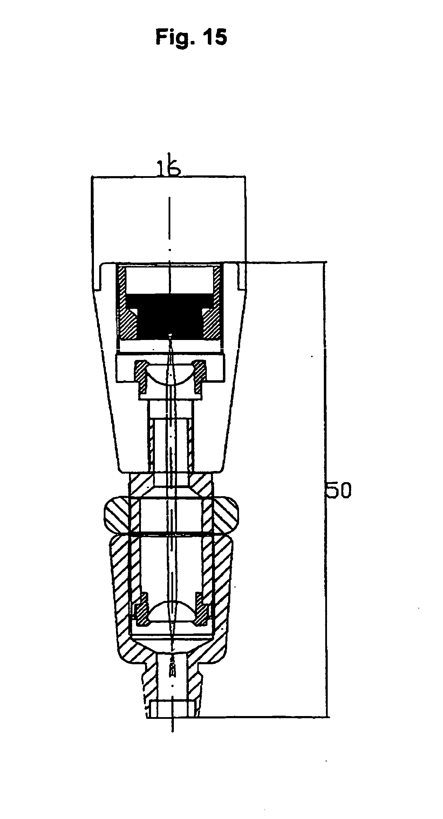

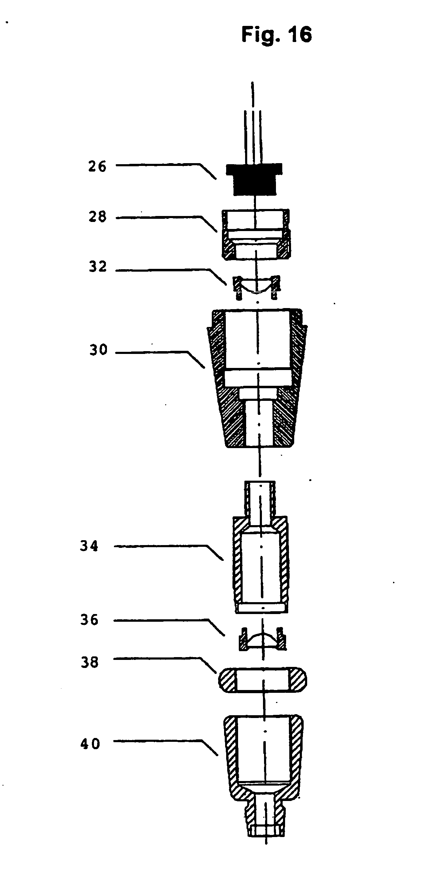

Das optische System des Bestrahlungsgeräts ist in Fig.14 bis 16 dargestellt, wobei gemäß Fig. 14 eine Strahldivergenz senkrecht von maximal 35 und gemäß Fig. 15 eine Strahldivergenz parallel von maximal 10˚ vorgegeben ist. Wie insbesondere aus der Explosionszeichnung gemäß Fig. 16 ersichtlich, ist die Laserdiode 26 in einer Gewindehülse 28 angeordnet. Es handelt sich um eine Multimode Laserdiode von 100 mW kontinuierlich für 670 nm einschließlich einer Monitordiode. In einem Kegelteil 30 befindet sich eine Objektivlinse 32, wobei ferner ein Linsenhalter 34 für eine weitere Objektivlinse 36 vorgesehen ist. Des Weiteren sind ein Verstellring 38 sowie ein Aufnahmekörper 40 vorgesehen.

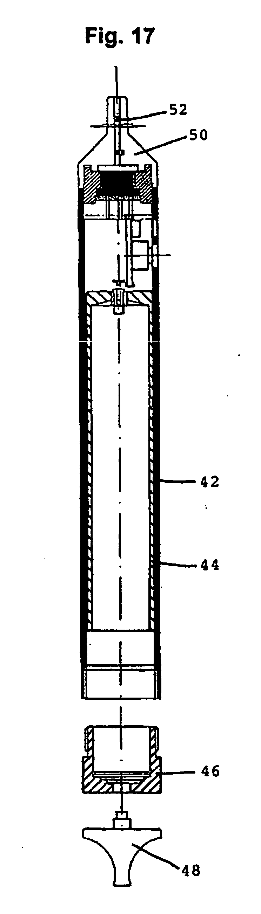

Fig. 17 zeigt das Bestrahlungsgerät ohne das optische System, wobei in einem Gehäuserohr 42 ein Batterierohr 44 für die zur Stromversorgung der Elektronik erforderlichen Batterien vorhanden ist. Am hinteren, gemäß Zeichnung unteren Ende des Gehäuserohres 42 ist eine Kappe 46 über eine Gewindeverbindung lösbar mit dem Gehäuserohr 42 verbunden bzw. verbindbar. Des Weiteren ist am hinteren Ende des Gehäuserohrs 42 bzw. der Kappe 46 ein Schlüsselschalter 48 vorgesehen, mittels welchem das Bestrahlungs- bzw. Lasergerät ein- oder ausschaltbar ist. Am vorderen Ende des Gehäuserohres, welches als Schutzgehäuse ausgebildet ist, ist ein Kopfteil 60 angeordnet, welches zur Aufnahme der Applikatoren und zur Lichtstrahlauskopplung durch die zentrale Bohrung 52 ausgebildet ist.

In Verbindung mit dem bevorzugt ausgebildeten Bestrahlungsgerät, welches auch als HEL-BOTherLite Laser bezeichnet wird, steht folgende Lichtleistung für die Belichtung zur Verfügung:

|  | Pocketsonde | Spotsonde |
|---|---|---|
| Abstrahlung | Radial, Bereich 250 - 280 Grad, insbesondere im wesentlichen 270 Grad frontal | Bereich 40-60 Grad, insbesondere im wesentlichen 50 Grad frontal |
| Leistungsdichte | >40mW/cm$^2$ | >40mW/cm$^2$ |

Die bevorzugt verwendete lichtaktivierbare Substanz ist eine sterile, isotonische, tiefblaue, geruchlose wässrige Flüssigkeit. Sie enthält Phenothiazin-5-ium, 3,7-bis (dimethylamino)-chlorid zur Einfärbung und Sensibilisierung von Mikroorganismen für die lethale photodynamische Therapie in Verbindung mit dem Bestrahlungsgerät.
1 ml der Lösung enthält:

- 1% Phenothiazin-5-ium, 3,7-bis (dimethylamino)-, chlorid
- Glucose zur Isotonisierung
- MHPC (Methylhydroxypropylcellulose) zur Einstellung der Viskosität
- Citrat zur Pufferung der Lösung.

Die Osmolarität entspricht ungefähr der von menschlichem Gewebe.

Die in Lösung mit der lichtaktivierbaren Substanz ist in einer Glasspritze verpackt und mit einem Stopfen aus Silikon verschlossen. Die Füllmenge beträgt 0,5 ml +/- 0,1 ml. Die Glasspritze ist einem Blister versiegelt und wird in einem validierten Sterilisationsprozess dampfsterilisiert. Die genannte Lösung ist zweckmäßig in fünf Blister mit einem Beipackzettel in einen Karton verpackt. 5 Kanülen 26G, die ebenfalls in je einem Blister verpackt und sterilisiert sind, sind beigepackt.

Die lichtaktivierbare Substanz dient im Rahmen der lethalen photodynamischen Lasertherapie zur Einfärbung und Sensibilisierung von Mikroorganismen bei lokalen oberflächlichen Infektionen, insbesondere im Mund-, Kiefer- und Gesichtsbereich. Durch die anschließende Belichtung mit dem Bestrahlungsgerät werden eingefärbte Mikroorganismen eliminiert und die natürliche Mundflora wiederhergestellt.

Die topische Applikation erfolgte im Bereich der Infektion ohne oder mit chirurgischer Eröffnung und Kürettage und paraläsional in folgender Weise: Der Patient spült mit Wasser zweimal für 20 Sekunden. An den zu behandelnden Flächen anhaftender Speichel oder Blut wird abgesaugt oder abgetupft, um eine Verdünnung von der photoaktiven Substanz zu verhindern.

Mit der Applikation der lichtaktivierbaren Substanz wird langsam begonnen, welche mittels Spritze flächendeckend auf die infizierten Gewebsareale aufgebracht wird. Die Menge ist unbedingt so zu wählen, dass der die lichtaktivierbare Substanz die Oberfläche der infizierten Bereiche möglichst dünn benetzt. Die vollständige Benetzung von Nischen und Taschen im Gewebe ist sicherzustellen. Gegebenenfalls bei komplexer Morphologie mit dem Luftbläser vorsichtig verteilen. Die Einwirkzeit der lichtaktivierbaren Substanz beträgt mindestens 60sec. Nach Spülung für mindestens 3 sec mit gleichzeitiger Absaugung überschüssiger Lösung (unbedingt Farbstoff-Depots entfernen!) mit dem Bestrahlungsgerät belichten. Wesentlich für die keimreduzierende Wirkung und damit für das Behandlungsergebnis, ist die richtige Dosierung der Energiezufuhr, die Belichtung.

Die Therapiekontrolle durch den Behandler enthält als wesentliche Größe für die Bestimmung der notwendigen Energiedichte (J/cm2) die Behandlungszeit entsprechend der vorgegebenen Behandlungsfläche. Mindestens 1 Minute Belichtungszeit mit dem Bestrahlungsgerät pro cm$^2$ oder pro Zahn sind einzuhalten.

Für die Berechnung der bestrahlten Fläche A einer Läsion mit einem Radius r während der Bestrahlung werden folgende Formeln eingesetzt :

a.: mit der Pocketsonde: Fläche des Defektes F = Breite B * Höhe*2 und damit pro Quadrat:

| Zähne | 1 | 2 | 3 | 4 | 5 | 6 | 7 | 8 |
|---|---|---|---|---|---|---|---|---|
| **Breite cm** | 0,6 | 1,2 | 1,8 | 2,4 | 3 | 3,6 | 4,2 | 4,8 |
| **Höhe cm** | 0,8 | 0,8 | 0.8 | 0,8 | 0,8 | 0,8 | 0,8 | 0,8 |
| **3DFaktor** | 2 | 2 | 2 | 2 | 2 | 2 | 2 | 2 |
| **Fläche cm$^2$** | 0,96 | 1,92 | 2,88 | 3,84 | 4,8 | 5,76 | 6,72 | 7,68 |
| **Leistung mW** | 50 | 50 | 50 | 50 | 50 | 50 | 50 | 50 |
| **Flächendichte W/cm$^2$** | 0,052 | 0,052 | 0,052 | 0,052 | 0,052 | 0,052 | 0,052 | 0,052 |
| **Belichtung sec** | 60 | 120 | 180 | 240 | 300 | 360 | 420 | 480 |
| **Energiedichte J/cm$^2$** | 3,125 | 3,125 | 3,13 | 3,125 | 3,125 | 3,125 | 3,125 | 3,125 |
| Belichtungsprotokoll für die Pocketsonde | | | | | | | | |

b: mit der Spotsonde: Fläche des Defektes F = ($\pi r^2$)

Die Leistungsdichte (FD) errechnet sich nach:

$$FD \ (Watt/cm^2) = Leistung \ (Watt)/ \ bestrahlte \ Fläche \ (cm2)$$

Die Energiedichte (ED) errechnet sich nach:

$$ED \ (Wattsec/cm^2) = Leistung \ (Watt)^*Zeit \ sec/ \ bestrahlte \ Fläche \ (cm^2)$$

Die jeweils gewählte Dosimetrie ist in der folgenden Tabelle zusammengestellt. In der Regel wird in einem Abstand von 0,55 cm bestrahlt und bei einer Leistungsdichte von 0,051 W/cm2, eine Gesamtdosis von 3 J/cm2 verwendet.

| Bestrahlungseinheit | 1 | 1 | 1 | 2 | 3 | 4 | 5 | 6 |
|---|---|---|---|---|---|---|---|---|
| **Durchmesser cm** | 1,8 | 0,9 | 1 | 1 | 1 | 1 | 1 | 1 |

(fortgesetzt)

| Bestrahlungseinheit | 1 | 1 | 1 | 2 | 3 | 4 | 5 | 6 |
|---|---|---|---|---|---|---|---|---|
| **Radius cm** | 0,9 | 0.45 | 0,5 | 0,5 | 0,5 | 0,5 | 0,5 | 0,5 |
| **Abstand cm** | 1 | 0,5 | 0,55 | 0.55 | 0,55 | 0.55 | 0,55 | 0.55 |
| **Fläche cm²** | 2,5434 | 0,6359 | 0,79 | 1,57 | 2,355 | 3,14 | 3,925 | 4,71 |
| **Leistung mW** | 40 | 40 | 40 | 40 | 40 | 40 | 40 | 40 |
| **Flächendichte W/cm²** | 0.016 | 0,063 | 0,051 | 0,051 | 0,051 | 0,051 | 0,051 | 0,051 |
| **Belichtung sec** | 60 | 60 | 60 | 120 | 180 | 240 | 300 | 360 |
| **Energiedichte J/cm²** | 0,94 | 3,77 | 3,06 | 3,06 | 3,06 | 3,06 | 3,06 | 3,06 |
| Belichtungsprotokoll für die Spotsonde | | | | | | | | |

Werden Bakterien durch lichtaktivierbare Substanzen bzw. Vitalfarbstoffe wie MB angefärbt und mit Licht geeigneter Wellenlänge bestrahlt, kann eine effektive phototoxische Wirkung induziert werden. Ungefärbten Zellen weisen keine toxischen Schädigungen auf. Von der photochemischen Abtötung möglicherweise pathogener Bakterien macht man in Pelztierfarmen oder Zoologischen Gärten Gebrauch, indem das Trinkwasser mit Methylenblau versetzt wird.

Als Photosensibilisator zur lokalen Behandlung von herpesbedingten Erkrankungen wird MB seit 25 Jahren eingesetzt. Die Dunkeltoxizität und Phototoxizität (PDT) von intratumoral appliziertem Methylenblau wurde experimentell an Kolontumoren untersucht. Eine Zerstörung dieser Tumoren nur durch Bestrahlung mit geringer Gesamtdosis (6 J/cm2) oder durch alleinige Verabreichung der Substanz (20 µg/ml) wurde nicht erreicht.

Die Gabe von MB kann zu systemischen Nebenwirkungen wie erhöhter Schweißbildung, Übelkeit und Erbrechen führen.

Bei oraler Verabreichung kann es zu gastrointestinalen Beschwerden und zu Dysurie kommen. In der Zubereitung der Lösung vorhandene Inhaltsstoffe sind:

| Wirk- und Hilfsstoffe | 1ml enthält [mg] |
|---|---|
| Methylenblau x 3 H2O | 10,00 |
| Trinatriumcitrat x 2 H2O | 0,433 |
| Citronensäure x 1H2O | 1,667 |
| MHPC | 10,00 |
| Natriumchlorid | 9,00 |
| Wasser für Injektionszwecke | 1000 |

Inhaltsstoffe der lichtaktivierbaren Substanz:

Nach der vorliegenden Biokompatibilitätsbewertung sind die in der Zubereitung verwendeten Substanzen hinsichtlich ihrer Biokompatibilität, Teratogenität und Mutagenität unter den gegebenen Anwendungsbedingungen als akzeptabel hinsichtlich des erstrebten Behandlungsziels beurteilt.

Mit der topischen Applikation des Photosensibilisators (PS) entfallen wesentliche Probleme der systemischen Anwendung eines Medikaments wie von Antibiotika, aber auch von systemisch eingesetzten PS, wie z. B Substanztoxizität und generalisierte, mehrwöchige Photosensibilisierung der Haut. Durch die topische Applikation lässt sich die Spezifität erhöhen, so dass gesundes Gewebe, z. B Schleimhaut in der Umgebung der Läsion, geschont wird.

Aufgrund der geringen Nebenwirkungen kann wiederholt behandelt werden. Die Therapie zeichnet sich weiterhin durch Nicht-Invasivität aus.

Die lokale Konzentration wird auch dadurch beeinflusst, dass nach lokaler Applikation des PS in der Regel eine erhöhte Speichelbildung der Mundschleimhaut induziert wird. Diese führt zur Verringerung der PS-Konzentration und vermindert die Penetration des Farbstoffs in die Läsion. Darüber hinaus können Speichelproteine durch unspezifische Bindung eine Inaktivierung des PS hervorrufen. Durch die Einbringung des PS in eine, insbesondere viskose Lösung wird erfindungsgemäß die Vermischung, Verdünnung und Reaktion mit Speichel für die Behandlungszeit verringert.

Nach der Einwirkzeit, die mit mindestens 60 sec angegeben ist, wird erfindungsgemäß der überschüssige PS entfernt, um die Lichttransparenz des behandelten Gewebes zu erhöhen.

Messungen zeigen, dass ein auf dem Gewebe stehender Flüssigkeitsfilm der Lösung mit der lichtaktivierenden Substanz von 100 $\mu$m die effektive Energiedichte um 97% verringert. Bei weiterer Verdopplung der Schichtdicke wird jeweils, gemäß dem Lambert-Beerschen Gesetz, das Licht weiter geschwächt. Somit ist eine therapeutisch wirksame Bestrahlung bei überschüssiger Lösung mit der lichtaktivierenden Substanz nicht möglich.

Für die effektive PDT-Applikation von Mundschleimhautläsionen wird eine Energiedichte von 50-100 J/cm2 empfohlen. Hierbei ist die Energiedosis der Art und der Lokalisation des Befundes anzupassen. Da die Mundschleimhaut generell sehr schmerzempfindlich ist, sollten Leistungsdichten oberhalb 150 mW/cm$^2$ vermieden werden. Leistungsdichten zwischen 200 und 500 mW/cm$^2$ können zu unspezifischen thermischen Gewebeschäden führen.

Um eine gleichmäßige Dosis im Bereich der Läsion zu erreichen, soll die Bestrahlungsfläche jeweils größer als die Fläche der Läsion gewählt werden.

Die Lichtdosis von ca. 100 J/cm$^2$ für die PDT lässt sich auf unterschiedliche Weise erzielen: Hohe Leistungsdichte und kurze Expositionszeiten oder niedrige Leistungsdichte und lange Expositionszeiten. Hohe Leistungen scheiden wegen der erwähnten thermischen Schäden aus. Andererseits lässt sich bei zu niedrigen Leistungsdichten ein photodynamischer Effekt auch bei entsprechend langen Bestrahlungszeiten nicht erzielen.

Methylenblaulösungen sind in der Lage, die Zahl aller untersuchten Mikrooganismen in den jeweiligen Kulturmedien zu reduzieren.

Methylenblau -Lösungen reduzieren in vitro nahezu alle grampositiven Bakterien bei einer Konzentration von 25-44 $\mu$mol. Die vollständige Reduzierung gramnegativer Keime verlangt 3- 30 fach höhere Konzentrationen. P.aeruginosa wurde bei einer Konzentration von 200 ~mol und einer Energiedichte von 100 mW/cm$^2$ um 3,5 log $_{10}$ CFU reduziert.

Die beobachtete Dunkeltoxizität war für Toluidinblau (TB) größer als für Methylenblau (MB). Dies stimmte mit dem Verteilungskoeffizient P überein, der für TB mit 0,33 und für MB mit 0,11 bestimmt worden war.

Da log P< 0 war sind somit beide Farbstoffe als hydrophil zu bezeichnen, und sollten, zumindest theoretisch die wassergefüllten Porin-Proteinkanäle gramnegativer Bakterien passieren können.

Während die Dunkeltoxizität für grampositive Bakterien kaum vom Typ abhängig war, war die Dunkeltoxizität für gramnegative Bakterien ganz deutlich vom Typ abhängig und zwar entsprechend dem transmembranen Permeabilitätskoeffizienten der äußeren Membran gramnegativer Bakterien.

Die Dunkeltoxizität war sowohl von der Konzentration als auch der Inkubationszeit vor Bestrahlung abhängig.

S.aureus wurde für die grampositive Gruppe als das resistentestes Bakterium identifiziert, dessen Vernichtung die höchsten Konzentrationen verlangte.

P .aeruginosa wurde für die gramnegative Gruppe als das resistentestes Bakterium identifiziert, dessen Vernichtung die höchsten Konzentrationen verlangte.

Bei gramnegativen Bakterien hängt die photodynamische Sensitivitätvon der transmembranen Permeabilität ab und die Hydrophilizität, die positive Ladung und das niedrige Molekulargewicht der Farbstoffmoleküle begünstigt die Wirksamkeit.

Für den vorliegenden Therapieansatz werden für die Behandlung mikrobiell infizierter Bereiche nur Einwirkungszeiten von 60 sec. mit anschließender Spülung vor der Belichtung vorgesehen.

Die gewähren Parameter der Therapie, insbesondere

- Konzentration der lichtaktivierenden Substanz in Lösung von 1%
- eine Energie von 2,4 J,
- einer Leistungsdichte von 50mW/cm$^2$
- und einer Energiedichte von 3 J/cm$^2$
- die Inkubationszeit von 60 sec mit anschließenden Ausspülen der Lösung

sind geeignet zur Gewährleistung eines sicheren Behandlungsergebnisses. Andererseits sind bei Einhaltung dieser Randbedingungen mögliche Risiken und Nebenwirkungen begrenzt und scheinen im Lichte der zu erwarteten positiven Aspekte für den Patienten nach entsprechender Aufklärung hinnehmbar.

Die PDT basiert auf einem photochemischen Prozess, bei dem Photosensibilisatoren (PS) mittels Laserstrahlung aktiviert werden und die eingestrahlte Energie so "portionieren", dass sie für die Bildung lokal toxisch wirkender Sauerstoffradikale zur Verfügung steht. Bei den gewählten Energie -und Leistungsdichten von 3 J/cm$^2$ bzw 50mW/cm$^2$ sind thermische Schädigungen des Gewebes sicher auszuschließen.

Die klinische Anwendung der PDT ist möglich, da die Farbstofflösungen Zellsysteme selektiv einfärben, andererseits die Wechselwirkung mit Epithel sehr begrenzt ist. Untersuchungen an normaler Mundschleimhaut zeigten dass die Eindringtiefe von MB -Lösungen nach 10 min Inkubationszeit nur auf die ersten 1-2 äußeren Zelllagen des Epithels begrenzt war. Weiterhin ist die Lebensdauer der aktiven Radikale und ihrer Vorstufen unter Mikrosekunden, so dass auch eine Ausdehnung der destruktiven Effekte ins gesunde Gewebe durch Diffusion praktisch ausgeschlossen ist, da die nötige Zeit nicht zur Verfügung steht.

Die Wirkung ist somit an die Anwesenheit der Farbstoffmoleküle des PS gebunden.

Die Wahl von beispielsweise Methylenblau als photodynamisch aktiver Substanz der Zubereitung begründet sich einerseits in der geringen Toxizität von Methylenblau unter den gewählten Behandlungsbedingungen, anderseits in der günstigen Lage des Absorptionsmaximums bei 664 nm:

- es stehen für diese Wellenlänge leistungsfähige und effiziente Dioden zur Erzeugung des Laserstrahles zur Verfügung

- es wird im Bereich des sichtbaren Lichtes behandelt, was entscheidend zur Sicherheit und Effizienz der Therapie beiträgt

- die Eindringtiefe des Lichtes ins Gewebe ist in diesem Wellenlängenbereich ausreichend tief um auch penetrierende Keimbesiedelung erreichen zu können

- die Singulettsauerstoffbildung ist der wesentliche Wirkmechanismus der Keimtötung wobei andererseits gesunde Zellen diese Radikale durch Katalasen abbauen

- können, andererseits auch durch die Wirksamkeit von Vitaminen wie Vitamin C und E ein gewisser Schutz besteht.

- anhand der Ergebnisse der spektralphotometrischen Untersuchungen MB mit und ohne Bestrahlungen erfolgte eine annähernd lineare Abnahme der Extinktion mit Zunahme der applizierten Energiedichte. Photobleaching mit Destruktion der Farbstoffmolekühle findet im wesentlichen Umfang bei Energiedichten statt, die um einen Faktor 7 über der therapeutisch aufgebrachten Energiedichte liegt .

- findet in diesem Bereich ein unterstützender photobiologischer Effekt statt, der die Gewebsregeneration fördert und die lokale Stoffwechsellage stabilisiert

- Methylenblau steht in reiner und dokumentierter Form zur Verfügung .die Verwendung in einer Zubereitung führt zu stabilen Lösungen

- diese Lösungen sind unter den Bedingungen des ärztlichen Umfeldes bei Anwendung angezeigter Sorgfalt einfach und sicher zu handhaben

- die bei der Anwendung anfallendes Abfälle sind relativ unschädlich

Durch die Abstimmung von Farbstoff und Lichtquelle steht ein therapeutisch effektives System zur Verfügung, welches sowohl gegen gram positive und gram negative Bakterien, als auch gegen Pilze wie candida albicans Wirkung zeigt und in der Lage ist, durch die Reduktion der Mikroorganismen die körpereigene Abwehr zeitlich begrenzt zu unterstützen um die klinische Symptomatik zu verbessern.

Mit den Applikatoren HELBO*PocketProbe* ist es möglich, erfindungsgemäß eine im wesentlichen im Bereich von 1 bis 7 J/cm$^2$, vorzugsweise von 2 bis 4 J/m$^2$, insbesondere im wesentlichen eine gleichmäßige Energiedichte von 3 J/cm$^2$ auch in den komplexen Arealen um und zwischen Zähnen im hinteren Bereichen der Mundhöhle zu applizieren.

Durch den Einsatz der photodynamischen Therapie können durch unterstützende photobiologische Effekte rasche Schmerzfreiheit und eine beschleunigte Wundheilung beobachtet werden.

Als Nebenwirkungen während der Therapie wurde gelegentliches Brennen beobachtet, das jedoch nach Ende der Behandlung rasch abklingt.

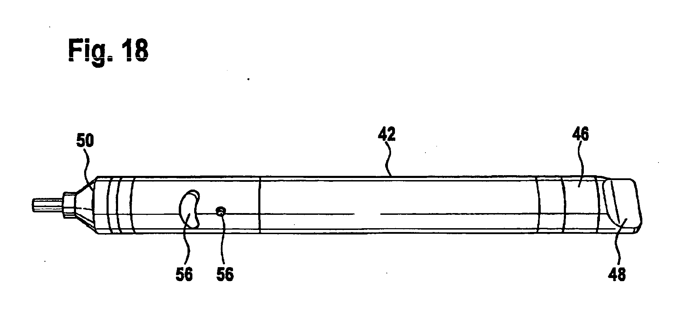

Eine besondere Ausgestaltung der Erfindung wird nachfolgend anhand der Fig. 18 bis 27 näher erläutert. Das Bestrahlungsgerät, welches nachfolgend auch als Therapielaser bezeichnet wird, dient zur photodynamischen Therapie (PDT) und ist insbesondere als Lasergerät ausgebildet Es enthält eine Sperreinrichtung, mittels welcher bei Ankoppeln eines Applikators, welcher einen Lichtleiter enthält, selbsttätig der Lichtweg freigegeben wird. Solange der Applikator und/oder der Lichtleiter an das Bestrahlungsgerät bzw. Lasergerät nicht angekoppelt ist, ist mittels der erfindungsgemäß ausgebildeten Sperreinrichtung der Austritt von Laserlicht aus dem Lasergerät blockiert. Die Sperreinrichtung enthält insbesondereeinen Veniegelungskörper, welcher derart ausgebildet und angeordnet ist, dass nur bei angekoppeltem Applikator bzw. Lichtleiter Laserlicht austreten kann. Der Verriegelungskörper der Sperreinrichtung ist in einer labilen Position angeordnet, aus welcher er ausschließlich bei ordnungsmäßerAnkopplung des Applikators bzw. Lichtleiters zur Freigabe des Lichtweges bringbar ist. Die Sperreinrichtung ist insbesondere in das Kopfteil des Bestrahlungsgeräts integriert. Alternativ kann die Sperreinrichtung an anderer Stelle des Bestrahlungsgeräts und/oder anders ausgebildet sein, und insbesondere in das optische System integriert sein. Das erfindungsgemäße Bestrahlungsgerät gewährleistet eine direkte Einkopplung des Lichtes aus der Diode bzw. Laserdiode in den ‚Lichtleiter derart, dass

1. geringe Verluste dadurch entstehen, dass eine Lichtleitfaser hoher numerischer Apertur zum Einsatz gelangt größer als 0,5, bevorzugt größer als 0,7, wodurch gewährleistet ist, dass eine relativ große Fläche gleichmäßig bestrahlt wird, da der Lichtstrahl bei Austritt aus der Faser stark öffnet,

2. nur bei Einbringung bzw. Ankopplung des Lichtleiters wird mittels der vorgebauten Sperreinrichtung der Lichtaustritt ermöglicht, und ansonsten wird ein direkter Lichtaustritt aus dem Lasergerät unterbunden. Somit kann das Lasergerät ohne Schutzbrille betrieben werden und ohne dass ein Laserschutzbeauftragter zu benennen ist, wie es bei der vorgesehenen Leistung des Lasergerätes grundsätzlich nötig wäre.

Aufgrund der hohen numerischen Apertur des zum Einsatz gelangenden Lichtleiters wird zum einen die erwähnte Streuwirkung beim Lichtaustritt bzw. Beleuchtung auf dem zu therapierenden Bereich erreicht und zum anderen auf der dem Lasergerät zugewandten Seite des Applikators bzw. des Lichtleiters eine Sammelwirkung derart sichergestellt, dass ein Linsensystem zwischen dem Laser bzw. der Diode und dem Lichtleiter entbehrlich ist.

Beim Einsatz des Bestrahlungs- bzw. Lasergerätes und/oderder Verwendung der Anordnung zur photodynamischen Therapie sind die nachfolgend aufgeführten Schritte von besonderer Bedeutung:

1. Die Lösung mit dem Photosensitizer wird zunächst in hoher Konzentration auf den zu therapierenden Bereich aufgebracht, damit jene möglichst schnell eindringt, insbesondere in den Plaque von Zähnen.

2. Zusätzlich wird bevorzugt eine Spülung mit einem Medium, insbesondere Wasser, möglichst geringer Ionenkonzentration durchgeführt, damit Bakterien und/oder Zellmembranen aufgrund des somit erzeugten osmotischen Druck-Gradienten gefährdet werden. Es sei festgehalten, dass beispielsweise eine physiologische Kochsalzlösung aufgrund der relativ hohen Ionenkonzentration ungünstig wäre. Der pH-Wert des Mediums wird vorteilhaft eher basisch vorgegeben. Bevorzugt wird ein pH-Wert von 7 bis 9 vorgegeben. Der Sauerstoffpartialdruck ist bevorzugt groß vorgegeben. Im Rahmen der Erfindung weist das Medium, insbesondere aufbereitetes Leitungswasser, zur Spülung einen Sauerstoffpartialdruck im Bereich von 4 bis 6 mg/l auf. Zweckmäßig ist das Medium angereichert mit molekularem Sauerstoff bis zu 14 mg/l. Weiterhin hat sich erfindungsgemäß eine Peroxidanreicherung als zweckmäßig erwiesen, und zwar bevorzugt als 0,5% bis 3%ige Wasserstoffperoxidlösung.

3. Aufgrund der vorherigen Spülung mit einem Medium geringer Konzentration wird bei der Bestrahlung mittels des Laserlichts eine optimierte Zellschädigung durchgeführt.

**Beschreibung des Bestrahlungs- bzw. Lasergerätes**

Das Gerät wird mit Batterien bzw. Akkumulatoren betrieben und verwendet als Licht- oder Strahlquelle einen Halbleiterlaser (Laserdiode), der kontinuierlich betrieben wird (cw). Fig. 18 zeigt das Bestrahlungs- bzw. Lasergerät von der Seite ohne Applikator Die Strahlquelle ist in einem zylindrischen, metallenen Schutzgehäuse 42 eingebaut. Die Länge des das Gehäuserohr 42 enthaltenden Schutzgehäuses beträgt etwa 124 mm, der Durchmesser etwa 16 mm. Am Ende des Schutzgehäuses 42, wo die Batterien bzw. Akkumulatoren einzulegen sind, wird eine Kontaktkappe 46 nach dem Einlegen der Batterien aufgeschraubt. Der betriebsbereite Zustand wird durch das Einsetzen des als Abschlusskappe geformten Schlüsselschalters 48 in die Kontaktkappe 46 erreicht. Der Betriebszustand wird durch verschiedenfarbige LEDs 54 angezeigt.

Am anderen Ende des Schutzgehäuses 42 ist das Kopfteil 50 auf das Schutzgehäuse 42 geschraubt und mit diesem verklebt, sodass der direkte Zugang zur Strahlquelle verhindert wird. Das Kopfteil 50 dient einerseits zur Aufnahme der Applikatoren und damit der Laserstrahlauskopplung, andererseits beinhaltet er den Verriegelungsmechanismus. Durch Drücken eines Tasters 56 wird schließlich der Halbleiterlaser aktiviert.

**Applikatoren**

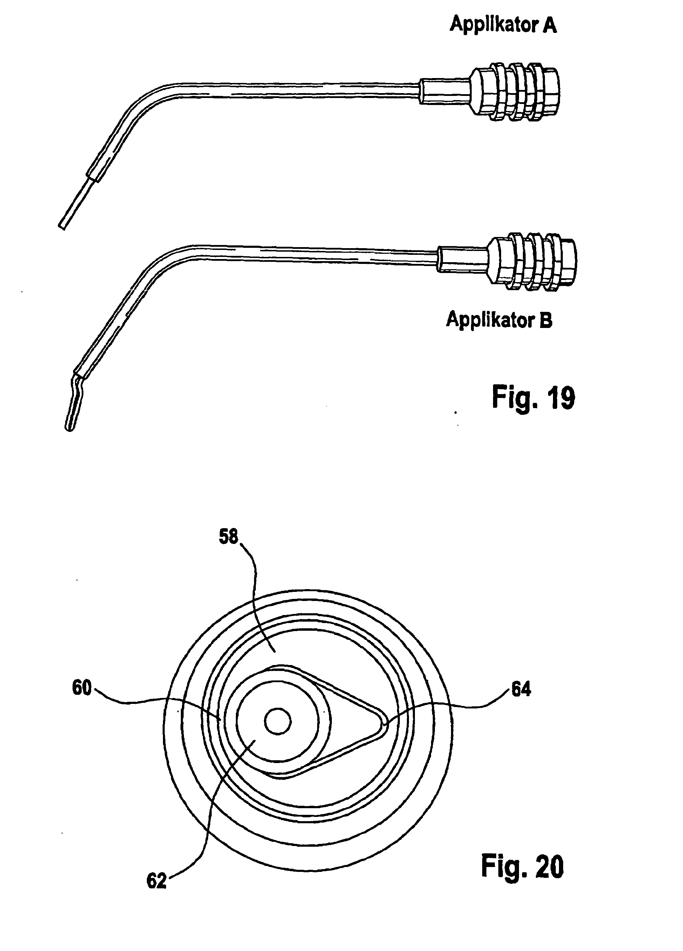

Fig. 19 zeigt zwei Ausführungsbeispiele von Applikatoren, wobei der im oberen Teil abgebildete Applikator A eine Pocketsonde ähnlich Fig. 2 ist, während der im unteren Teil abgebildete Applikator B eine Flächensonde ähnlich Fig. 3 ist. Beide Applikatoren bestehen aus einem transparenten Kunststofflichtleiter mit einem Durchmesser von etwa 1 mm, haben einen Luerstecker zur Aufnahme am Kopf des Lasergerätes, sind gebogen und mit einem weißen Schutzmantel zwischen Luerstecker und Strahlaustrittsende umgeben. Das Lichtleiterende (ohne Schutzmantel) beim Luerstecker wird in den Kopf des Schutzgehäuses gesteckt.

Applikator A weist eine leicht konische Spitze auf, deren Oberfläche auf den letzten 5 mm aufgeraut ist. Die raue Oberfläche bewirkt, dass das Laserlicht in nahezu alle Raumrichtungen abgestrahlt wird, wobei in axiale Richtung des Lichtleiters die meiste Energie abgegeben wird. Applikator B hat eine ebene Stirnfläche als Austrittsfläche für das laserlicht. Zusätzlich ist eine Drahtschleife am Lichtleiterende als Abstandshalter eingebaut. Im Gegensatz zu Applikator A weist das Laserlicht eine divergierende, konische Abstrahlcharakteristik auf, wodurch in axiale Richtung mehr Energie abgegeben wird. Für alle weiteren Messungen wurde daher Applikator B verwendet, da er aus Sicht der Lasersicherheit das größere Gefahrenpotential beinhaltet.

**Sperreinrichtung / Verriegelungsmechanismus**

Der in Fig. 20 dargestellte Verriegelungsmechanismus enthält einen im Querschnitt "H"-förmigen, rotationssymmetrischen Verriegelungskörper 58, der in der Mitte ein Loch 60 mit 1.01 +0,02 mm Durchmesser hat. In der Ausnehmung des "H's", das dem Strahlaustritt abgewandt ist, ist die Laserdiode positioniert und emittiert Licht im aktivierten Zustand durch das Loch 60 im Verriegelungskörper 58 in Richtung Strahlaustritt.

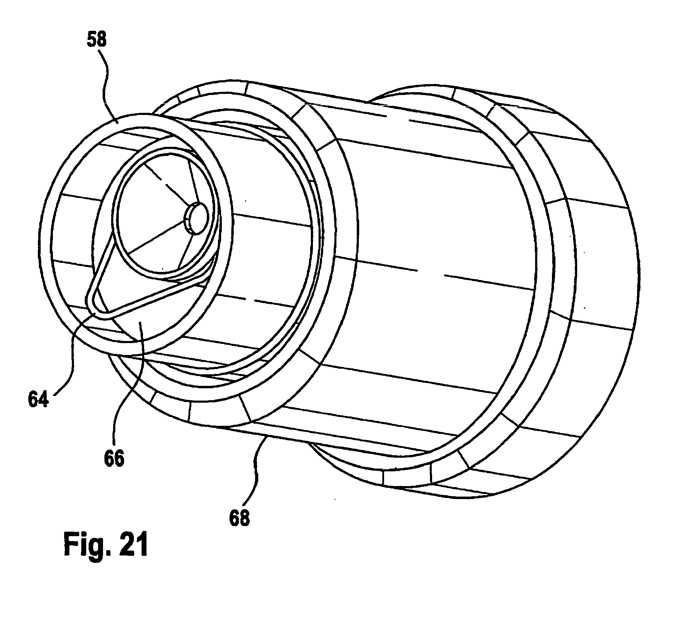

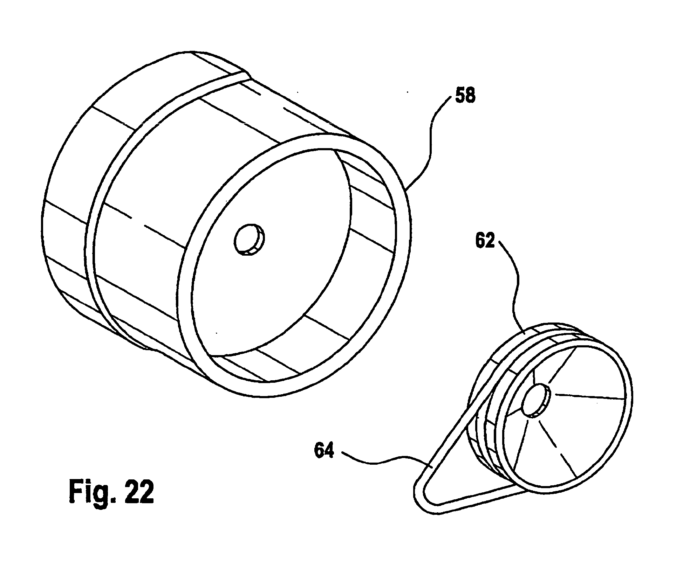

In der Vertiefung des "H's", die dem Strahlaustritt zugewandt ist, befindet sich eine runde Verriegelungsscheibe 62, ebenfalls mit einem in der Mitte befindlichen Loch mit 1,01 +0,02 mm Durchmesser. Der Außendurchmesser dieser Scheibe 62 ist wesentlich kleiner als der Innendurchmesser des Verriegelungskörpers 58, sodass gemäß Fig. 20 sich die Scheibe in der Vertiefung verschieben läßt. Die Verriegelungsscheibe wird jedoch mittels einer Drahtfeder (Verriegelungsfeder) azentrisch gehalten. Damit deckt diese Scheibe 62 das Loch des Verriegelungskörpers ab und das Laserlicht kann nicht austreten. Die Drahtfeder 64 wird in einer Nut am Umfang der Verriegelungsscheibe geführt. Wie aus Fig. 21 und der explosionsartigen Darstellung gemäß Fig. 22 des Verriegelungskörpers 58 und der Verriegelungsscheibe 62 ersichtlich, ist der Verriegelungskörper 58, in dessen Ausnehmung die Verriegelungsschreibe bewegbar angeordnet ist, in einem Diodenhalter 68 angeordnet. In der explosionsartigen Darstellung der Fig. 22 sind die beiden genannten Löcher des Verriegelungskörpers 58 und der Verriegelungsscheibe 62 gut zu erkennen.

Erst durch die Einführung des Lichtleiters des Applikators bis zum Anschlag wird die Verriegelungsscheibe 62 in die zentrale Position gedrückt, so dass sich das Loch der Verriegelungsscheibe 62 und das Loch des Verriegelungskörpers 58 überdecken und das Laserlicht in den Lichtleiter eingekoppelt werden kann. Wird der Lichtleiter herausgezogen, dann drückt die Drahtfeder 64 die Verriegelungsscheibe 62 in die Ausgangsposition zurück, und das Laserlicht wird abgeblockt. Anstelle der hier dargestellten Drahtfeder 64 können im Rahmen der Erfindung auch andere Rückführelemente vorgesehen sein, um den Austritt von Laserlicht nur dann zu ermöglichen, wenn der Applikator und insbesondere dessen Lichtleiterende ordnungsgemäß mit dem Bestrahlungsgerät verbunden ist.

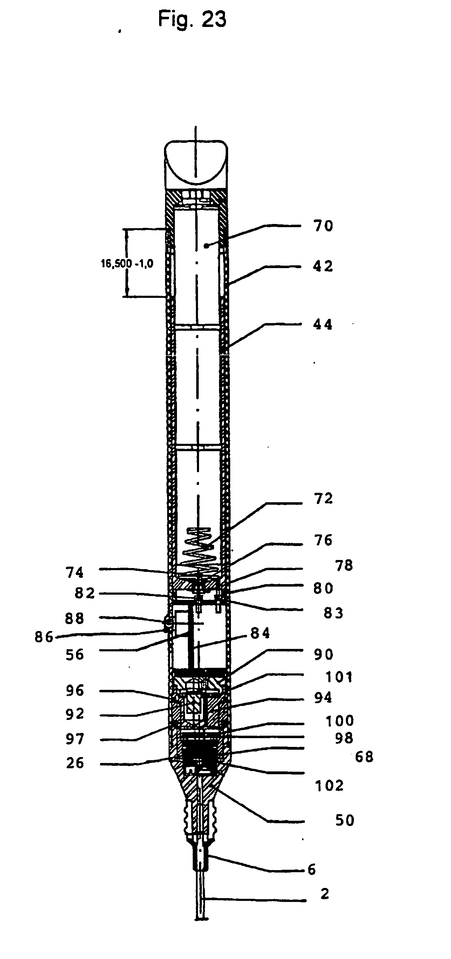

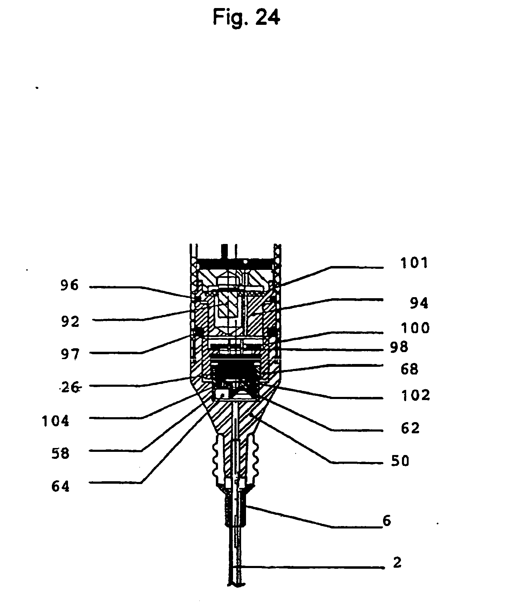

Fig. 23 zeigt einen Schnitt durch das Bestrahlungsgerät und Fig. 24 zeigt vergrößert dessen Vorderteil. Dieses Bestrahlungsgerät entspricht grundsätzlich dem oben bereits Erläuterten und enthält zusätzlich die Sperreinrichtung bzw. den Verriegelungsmechanismus. In dem vom Gehäuserohr 42 umgebenen Batterierohr 44 sind drei Batterien 70 angeordnet, welche mittels einer Batteriefeder 72 beaufschlagt sind. Zur Kontaktierung ist eine Steckerbuchse 74 in einer Buchsenführung 76 angeordnet, wobei ferner eine Scheibe 78, eine Distanzscheibe 80 vorhanden sind. Des Weiteren sind zwei Steckerstifte 82, 83 zur Kontaktierung mit einer Elektronik oder Elektronikplatine 84 vorgesehen. Auf der Elektronikplatine 84 ist der von außen betätigbare Taster 56 angeordnet, wobei insbesondere zur Abdichtung einer Tasterfolie 86 zusammen mit einer Kugel 88 vorgesehen sind. Nach vorn anschließend an den Innenbereich mit der Elektronik 84 und abgetrennt mittels einer hinteren Isolierung 90 ist ein Bereich mit einem Schlagbolzen 92 vorgesehen, wobei ferner eine vordere Isolierung 94 vorhanden ist. Innerhalb des Gehäuserohres 42 sind ferner zwei O-Ringe 96, 97 angeordnet. Am vorderen Ende des Gehäuserohres 42 ist das Kopfteil 60 angeordnet, dessen vorderes Ende in den Verbindungskörper bzw. Luerstecker 6 des hier nicht weiter dargestellten Applikators eingreift. Im Kopfteil 50 ist der bereits erläuterte Diodenhalter 68 mit der Laserdiode 26 angeordnet, wobei nach hinten zum Batterierohr 44 hin eine Isolierscheibe 98 sowie ein Print 100 für die Diode einschließlich zur Kontaktierung erforderlicher Drähte vorgesehen ist. Zusätzlich ist in Strahlungsrichtung vor der Laserdiode eine Schutzfolie 102 vorgesehen, mittels welcher in bevorzugter Weise die Laserdiode gegen äußere Einwirkungen geschützt wird, wobei zusätzlich ein 0-Ring 104 vorgesehen ist. Des Weiteren sind in der inneren Ausnehmung des Kopfteils 50 der bereits erläuterte Verriegelungskörper 58, die Verriegelungsscheibe 62 sowie die Verriegelungsfeder 64 angeordnet.

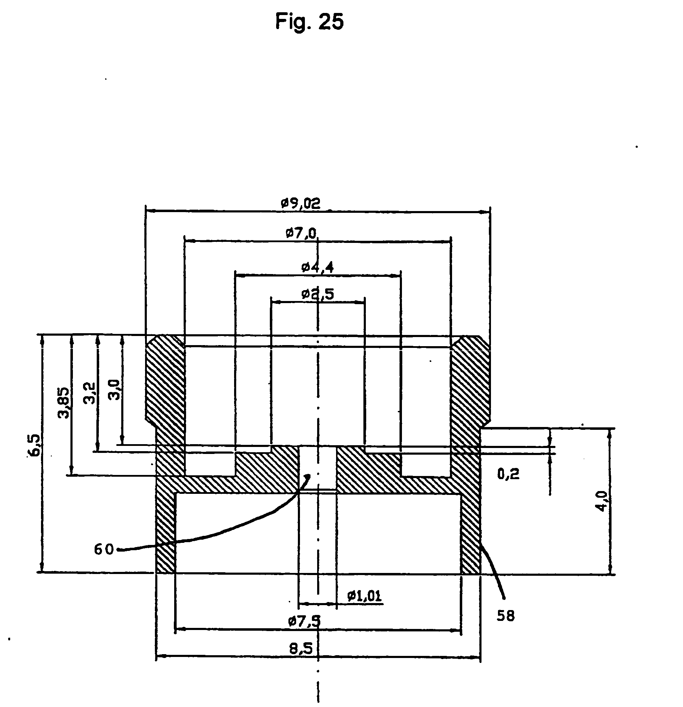

Fig. 25 zeigt einen Schnitt in einer axialen Schnittebene durch den Verriegelungskörper 58, wobei dessen H-förmige Querschnittsfläche gut zu erkennen ist. Es versteht sich, dass die für die besondere Ausgestaltung angegebenen Abmessungen in Millimeter auch abweichend vorgegeben werden können.

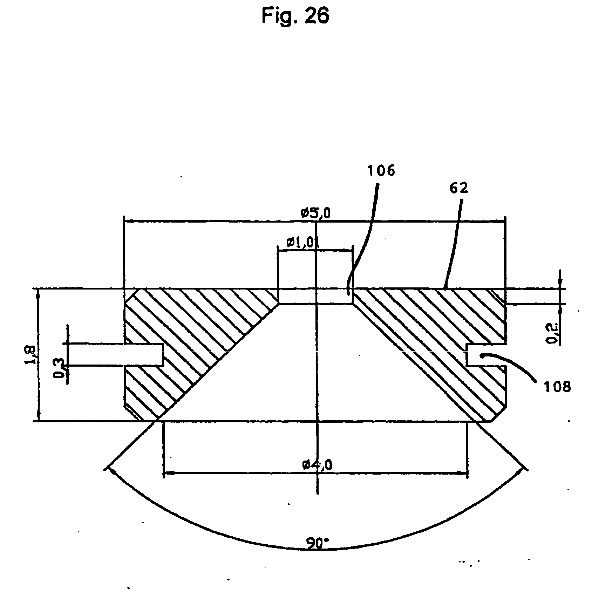

In Fig. 26 ist ein Schnitt in einer axialen Schnittebene durch die Verriegelungsscheibe 62 dargestellt, welche in der Mitte das bereits erwähnte Loch 106 enthält Am Außenumfang weist die Verriegelungscheibe eine Nut 108 auf, in welche die Draht- bzw. Verriegelungsfeder eingreift.

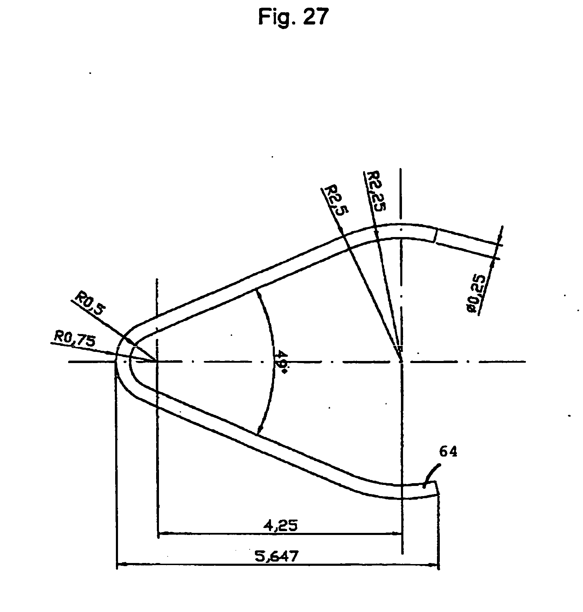

Schließlich zeigt Fig. 27 eine Ansicht der Verriegelungsfeder 64. Es bedarf keiner besonderen Hervorhebung, dass für andere Ausführungsformen der Sperreinrichtung die Abmessungen der Verriegelungsfeder ebenso wie die der übrigen Bauteile abweichend vorgegeben sein können.

**Funktionsbeschreibung Sperreinrichtung**

Die Sperreinrichtung enthält folgende Einzelteile:

- Verriegelungsplättchen / Verriegelungsscheibe
- Feder Sperreinrichtung / Verriegelungsfeder
- Verriegelungshalter / Verriegelungskörper
- Diodenhalter / Diodenhalter
- Diode
- Kopf R60 / Kopf f. Luerstecker

Die Teile werden wie folgt angeordnet:

Die Diode wird im Diodenhalter eingebracht und mit der ESD Platine von Hinten fixiert. Die Diode sitzt nun im Diodenhalter. Die Diode wird über den Innendurchmesser des Diodenhalters ausgerichtet. Nun kann der Verriegelungshalter auf die Diode aufgeschoben werden. Der Verriegelungshalter wird über den Innendurchmesser des Diodenhalters positioniert. Der O-Ring im Verriegelungshalter fängt leichte Stöße in Längsrichtung ab.

Die Verriegelungsfeder wird in die Nut des Verriegelungsplättchen eingebracht. Diese Anordnung wird in die Öffnung des Verriegelungshalters eingelegt. Der Kopf wird über den Verriegelungshalter und den Diodenhalter geschoben.

Funktionsbeschreibung:

Der Lichtleiter wird über die 1,5 mm Bohrung des Kopfes in den Lichtleitergang eingeführt. Nach ca. 7mm wird der Lichtleiter über eine Verringerung des Durchmessers auf 1,1 mm positioniert. Der Lichtleiter wird dann bis zum Verriegelungsplättchen vorgeschoben.
Das Verriegelungsplättchen wird durch die Vorspannung der Feder auf den Innenrand des Verriegelungshalters gepresst. Das Plättchen ist dadurch immer dezentriert und verdeckt die 1,01 mm Bohrung des Verriegelungshalters, dadurch kann keine Strahlung austreten.
Der Lichtleiter trifft auf dem Konus des Verriegelungsplättchens auf und drückt dieses entgegen der Federkraft in die Mitte des Verriegelungshatters. Nachdem das Verriegelungsplättchen zentriert wurde, wird die 1,01 mm Bohrung des Verriegelungshalters freigegeben und der Lichtleiter wird durch diese Öffnung in den Einkoppelbereich vorgeschoben.
Der Lichtleiter hat den Einkoppelbereich erreicht wenn der Luerstecker den Anschlag am Kopf erreicht hat.

Beim Herausziehen des Lichtleiters schiebt die Verriegelungsfeder das Plättchen wieder in die dezentrale Position, wodurch die 1,01 mm Bohrung wieder verschlossen wird.

Diskussion der Sicherheit des Verriegelungsmechanismus

Der Federdraht der Verriegelungsfeder hat einen Durchmesser von 0,25mm. Die Nut hat 0,3 mm. Da der Draht eine Runde Form besitzt, kann er sich in der Nut nicht verklemmen. Dadurch wird die Sperreinrichtung sicher. Die Kanten des Verriegelungsplättchens sind durch Gleitschleifen auf 0,2 mm gebrochen.

Die Feder presst das Verriegelungsplättchen immer nach außen. Durch die Lage des Lasers im Betrieb ist auch ohne Verriegelungsfeder eine Verriegelung gegeben. Das Verriegelungsplättchen wird durch sein Gewicht nach unten gezogen, wodurch die 1,01 mm Bohrung verschlossen wird. Durch die spezielle Formgebung der Feder kann ein herausspringen der Feder aus der Nut auch ausgeschlossen werden. Die Federform umschließt das Verriegelungsplättchen und sichert es somit.

Eine Neigung des Plättchens hat durch den kleinen Winkel keine Wirkung auf den Verriegelungsmechanismus. Das Plättchen richtet sich beim nächsten Lichtleitereinschub wieder in seine Position aus.

Verschmutzungen des Verriegelungsmechanismus haben keinen Einfuß auf die Funktion der Verriegelung. Es ist nur mit Abriebsstaub des Lichtleiters zu rechnen. Durch die flexible Ausführung des Lichtleiters ist nur mit geringen Abrieb zu rechnen. Bei der jährlichen Überprüfung kann eine Säuberung des Mechanismus einfach durchgeführt werden. Fremdkörper können nur in der Größe von 1,1 mm auftreten, da diese Größe durch die Durchführungsbohrung im Kopf begrenzt wird.

Eventuelles Nachlassen der Federkraft, wird durch Tests nachkontrolliert. Auch Veränderungen durch Fall auf den Boden werden nachgemessen.

**EP 2 204 218 B1**

**Bezugszeichen**

| | |
|---|---|
| 2 | Lichtleiter |
| 4 | Schutzmantel von 2 |
| 6 | Verbindungskörper / Luerstecker |
| 8 | Lichtleiterende |
| 10 | gebogener Bereich von 2 |
| 12 | Spitze von 2 |
| 14 | Applikationsspritze für den Photosensitizer |
| 16 | Bereich |
| 18 | freies Ende von 2 |
| 20 | Abstandshalter |
| 22 | angeschliffenes freies Ende von 2 |
| 24 | Spitze von 22 |
| 26 | Lichtquelle / Laserdiode / Halbleiterlaser |
| 28 | Gewindehülse |
| 30 | Kegelteil |
| 32 | Objektivlinse |
| 34 | Linsenhalter |
| 36 | zweite Objektivlinse |
| 38 | Verstellring |
| 40 | Aufnahmekörper |
| 42 | Gehäuserohr / Schutzgehäuse |
| 44 | Batterierohr |
| 46 | Kontaktkappe |
| 48 | Schlüsselhalter |
| 50 | Kopfteil |
| 52 | zentrale Bohrung in 50 |
| 54 | Betriebsanzeige / LEDs |
| 56 | Taste |
| 58 | Verriegelungskörper |
| 60 | Loch in 58 |
| 62 | Verriegelungsscheibe |
| 64 | Verriegelungsfeder / Drahtfeder |
| 66 | Ausnehmung |
| 68 | Diodenhalter |
| 70 | Batterie |
| 72 | Batteriefeder |
| 74 | Steckerbuchse |
| 76 | Buchsenführung |
| 78 | Scheibe |
| 80 | Distanzscheibe |
| 82, 83 | Steckerstift |
| 84 | Elektronikplatine |
| 86 | Tasterfolie |
| 88 | Kugel |
| 90 | hintere Isolierung |
| 92 | Schlagbolzen |
| 94 | vordere Isolierung |
| 96, 97 | O-Ring |
| 98 | Isolierscheibe |
| 100 | Print |
| 102 | Schutzfolie |
| 104 | O-Ring |
| 106 | Loch in 62 |
| 108 | Nut in 62 |

**Patentansprüche**

1. Anordnung zur Reduktion von Mikroorganismen, enthaltend ein Bestrahlungsgerät, welches eine Lichtquelle (26) und einen ersten Verbindungskörper (50) aufweist, sowie wenigstens einen Applikator, welcher einen von einem Schutzmantel (4) umgebenen Lichtleiter (2) und einen zweiten Verbindungskörper (6) enthält, mittels welchem der Applikator mit dem Bestrahlungsgerät über dessen ersten Verbindungskörper (50) lösbar verbunden ist, wobei die Verbindungskörper (50, 6) miteinander korrespondieren und ineinander greifen derart, dass das Licht der Lichtquelle (26) durch den Lichtleiter (2) auf einen zu therapierenden Bereich strahlbar ist, auf welchen zur Therapie eine Licht aktivierbare Substanz aufgebracht ist,

   **gekennzeichnet durch** Einsetzbarkeit zur Therapie eines Bereichs der Mundhöhle derart, dass der Lichtleiter (2) eine numerische Apertur größer 0,5 aufweist und ein vom Schutzmantel (4) freies Ende (18, 22) aufweist, wobei der aus dem Lichtleiter (2) austretende Lichtstrahl stark öffnet,

   dass die Abmessungen des Applikators derart vorgeben sind, dass er in die Mundhöhle einführbar ist und als Pocketsonde mit angeschliffener konischer Spitze oder als Flächensonde ausgebildet ist,

   dass das Bestrahlungsgerät ein Gehäuserohr (42) und ein an dessen vorderen Ende angeordnetes Kopfteil (50) aufweist, welches als der erste Verbindungskörper ausgebildet ist,

   dass im vorderen Teil des Gehäuserohrs (42) die Lichtquelle (26) und eine Elektronikplatine (84) mit einer von außen betätigbaren Taste (56) angeordnet sind,

   und dass im Gehäuserohr (42) ferner Batterien (70) oder Akkumulatoren zur Stromversorgung der Elektronik für die Lichtquelle (26) vorgesehen sind, wobei am hinteren Ende des Gehäuserohrs (42) eine Kappe (46) über eine Gewindeverbindung lösbar mit diesem verbunden ist.

2. Anordnung nach Anspruch 1, **dadurch gekennzeichnet, dass** von der Lichtquelle (26), welche bevorzugt als Laserdiode ausgebildet ist, in den Applikator entweder eine direkte Lichteinkopplung oder eine Einkopplung über ein Linsenpaket (32, 36) erfolgt.

3. Anordnung nach Anspruch 1 oder 2, **dadurch gekennzeichnet, dass** das Kopfteil (50) zur Aufnahme der Applikatoren und zur Lichtauskopplung durch eine zentrale Bohrung (52) ausgebildet ist und/oder dass ein vorderes Ende des Kopfteils (50) in den Verbindungskörper (6) des Applikators eingreift.

4. Anordnung nach einem der Ansprüche 1 bis 3, **dadurch gekennzeichnet, dass** die zentrale Bohrung (52) zur Aufnahme und/oder Zentrierung des Lichtleiterendes (8) des Applikators ausgebildet ist.

5. Anordnung nach einem der Ansprüche 1 bis 4, **dadurch gekennzeichnet, dass** am hinteren Ende des Gehäuserohrs (42) oder der Kappe (46) ein Schlüsselschalter (48) vorgesehen ist, mittels welchem das Bestrahlungsgerät ein- oder ausschaltbar ist.

6. Anordnung nach einem der Ansprüche 1 bis 5, **dadurch gekennzeichnet, dass** die Applikatoren als Einmaloptiken für eine einmalige Benutzung ausgebildet sind.

7. Anordnung nach einem der Ansprüche 1 bis 6, **dadurch gekennzeichnet, dass** das Gehäuserohr (42) eine Betriebsanzeige (54) aufweist.

8. Anordnung nach einem der Ansprüche 1 bis 7, **dadurch gekennzeichnet, dass** im Gehäuserohr (42) im Strahlengang vor der Lichtquelle (26) eine Schutzfolie (102) vorgesehen ist.

9. Anordnung nach einem der Ansprüche 1 bis 8, **dadurch gekennzeichnet, dass** das Kopfteil (50) einen Verriegelungsmechanismus derart beinhaltet, dass aus dem Bestrahlungsgerät Licht nur bei vollständiger Verbindung des Applikators mit dem Bestrahlungsgerät und/oder bei vollständig in das Bestrahlungsgerät eingebrachtem Lichtleiter (2) des Applikators austreten kann.

10. Anordnung nach Anspruch 9, **dadurch gekennzeichnet, dass** der Verriegelungsmechanismus einen mit dem Bestrahlungsgerät fest verbundenen Verriegelungskörper (58) mit einem im Strahlengang der Laserdiode (26) angeordneten Loch (60) enthält und dass eine bezüglich des Verriegelungskörpers (8) bewegbar angeordnete Verriegelungsscheibe (62) mit einem Loch (106) vorgesehen ist, wobei der Strahlengang der Lichtquelle (26) mittels der Verriegelungsscheibe (62) solange unterbrochen ist, als der Applikator mit dem Bestrahlungsgerät nicht verbunden ist, und wobei nach Herstellung einer Verbindung des Applikators mit dem Bestrahlungsgerät der Strahlengang von der Laserdiode (26) in den Lichtleiter (2) des Applikators freigegeben ist und/oder das in der Verriegelungsscheibe (62) vorgesehene Loch mit dem im Strahlengang befindlichen Loch (60) des Verriegelungskörpers (58) fluchtet.

11. Anordnung nach einem der Ansprüche 1 bis 10, **dadurch gekennzeichnet, dass** der Verbindungskörper (6) des Applikators als ein Steck- oder Schraubverschluß mit einem Tiefenanschlag ausgebildet ist.

12. Anordnung nach einem der Ansprüche 1 bis 11, **dadurch gekennzeichnet, dass** der Lichleiter (2) des Applikators durch den Verbindungskörper (6) des Applikators hindurchgeführt ist und mit seinem Lichtleiterende (8) in einer vorgegebenen Länge nach hinten aus dem Verbindungskörper (6) herausragt und bei hergestellter Verbindung mit dem Bestrahlungsgerät in dessen Kopfteil (50) eingreift.

13. Anordnung nach einem der Ansprüche 2 bis 12, **dadurch gekennzeichnet, dass** die Wellenlänge der Laserdiode 660 nm beträgt.

**Claims**

1. Assembly for reducing microorganisms, comprising an irradiation device which has a light source (26) and a first connection body (50), as well as at least one applicator, which comprises a light guide (2), surrounded by a protective casing (4), and a second connection body (6), by means of which the applicator is detachably connected with this irradiation device via its first connection body (50), whereby the connection bodies (50, 6) correspond with each other and interconnect in such a way that the light from the light source (26) can be directed through the light guide (2) onto an area to be treated, on which a substance activated by light is applied for treatment, **characterised by** being able to be used to treat an area of the oral cavity in such a way that the light guide (2) has a numerical aperture greater than 0.5 and the end (18, 22) is free of the protective casing (4), whereby the light beam emerging from the light guide (2) opens out greatly,
in that the dimensions of the applicator are predetermined in such a way that it can be inserted into the oral cavity and is designed as a pocket probe with a ground conical tip or as a flat probe,
in that the irradiation device has a housing tube (42) and, arranged at its front end, a head section (50) which is designed as the first connection body,
in that in the front section of the housing tube (42) a light source (26) and an electronic board (84) with an externally operated button (56) are arranged,
and in that in the housing tube (42) batteries (70) or rechargeable batteries are provided for supplying power to the electronics of the light source (26), whereby at the rear end of the housing tube (42) a cap (46) is detachably connected thereto by means of a screw-type connection.

2. Assembly in accordance with claim 1 **characterised in that** from the light source (26), which is preferably in the form of a laser diode, direct light injection or injection via a lens package (32, 36) takes place into the applicator.

3. Assembly in accordance with claim 1 or 2 **characterised in that** the head section (50) is designed to accommodate the applicator and for light injection through a central boring (52) and/or **in that** a front end of the head section (50) engages in the connection body (6) of the applicator.

4. Assembly in accordance with any one of claims 1 to 3 **characterised in that** the central boring (52) is designed to accommodate and/or centre the light guide end (8) of the applicator.

5. Assembly in accordance with any one of claims 1 to 4 **characterised in that** at the rear end of the housing tube (42) or the cap (46) a key switch (48) is provided with which the irradiation device can be switched on and off.

6. Assembly in accordance with any one of claims 1 to 5 **characterised in that** the applicators are designed as disposable optical devices for single use.

7. Assembly in accordance with any one of claims 1 to 6 **characterised in** the casing tube (42) has an operating display (54).

8. Assembly in accordance with any one of claims 1 to 7 **characterised in that** in the housing tube (42) a protective foil (102) is provided in the beam path before the light source (26).

9. Assembly in accordance with any one of claims 1 to 8 **characterised in that** the head section (50) contains a locking mechanism in such a way that light can only emerge from the irradiation device when the applicator is fully connected to the irradiation device and/or when the light guide (2) is fully introduced in the irradiation device.

10. Assembly in accordance with claim 9, **characterised in that** the locking mechanism has a locking body (58) which is firmly connected to the irradiation device and has a hole (60) arranged in the beam path of the laser diode (26) and **in that** a locking disc (62) with a hole (106) is envisaged which is movable with regard to the locking body (8), whereby the beam path of the light source (26) is interrupted by the locking disc (62) for as long as the application is not connected to the irradiation device, and whereby after connecting the applicator to the irradiation device the beam path from the laser diode (26) in released into the light guide (2) of the applicator and/or the hole in the locking disc (62) is aligned with the hole (60) of the locking body (58) located in the beam path.

11. Assembly in accordance with any one of claims 1 to 10 **characterised in that** the connection body (6) of the applicator is designed as a plug or screw connection with a depth stop.

12. Assembly in accordance with any one of claims 1 to 11 **characterised in that** the light guide (2) of the applicator is passed through the connection body (6) of the applicator and the light guide end (8) projects backward for a predetermined distance out of the connection body (6) and, when connection to the irradiation device takes place, engages in the latter's head section (50).

13. Assembly in accordance with any one of claims 2 to 12 **characterised in that** the wavelength of the laser diode is 660 nm.


**Revendications**

1. Système pour la réduction de micro-organismes, comprenant un appareil d'irradiation présentant une source de lumière (26) et un premier corps de connexion (50), ainsi qu'au moins un applicateur comprenant un guide de lumière (2) entouré d'une gaine protectrice (4) et un deuxième corps de connexion (6) au moyen duquel l'applicateur est connecté de façon amovible à l'appareil d'irradiation par l'intermédiaire du premier corps de connexion (50) de celui-ci, lesdits corps de connexion (50, 6) correspondant l'un à l'autre et s'engageant l'un dans l'autre de telle sorte que la lumière de la source de lumière (26) peut être amenée à rayonner travers le guide de lumière (2) sur une zone à traiter sur laquelle une substance pouvant être activée par la lumière est appliquée en vue d'un traitement, **caractérisé en ce qu'**il peut être mis en oeuvre pour un traitement d'une zone de la cavité buccale de telle sorte que le guide de lumière (2) présente une ouverture numérique supérieure à 0,5 et une extrémité (18, 22) sans gaine protectrice (4), le rayon de lumière sortant du guide de lumière (2) s'ouvrant largement,
**en ce que** les dimensions de l'applicateur sont spécifiées de telle sorte qu'il peut être introduit dans la cavité buccale et est réalisé sous forme de sonde de poche à pointe conique affûtée ou sous forme de sonde de surface,
**en ce que** l'appareil d'irradiation présente un tube formant boîtier (42) et une partie de tête (50) disposée au niveau de l'extrémité avant de celui-ci et qui est réalisée en tant que premier corps de connexion,
**en ce que** la source de lumière (26) et une platine électronique (84) avec une touche (56) pouvant être actionnée depuis l'extérieur sont disposées dans la partie avant du tube formant boîtier (42), et
**en ce que** dans le tube formant boîtier (42), en outre des batteries (70) ou des accumulateurs sont prévus pour l'alimentation électrique de l'électronique pour la source de lumière (26), dans lequel, à l'extrémité arrière du tube formant boîtier (42), un capuchon (46) est connecté à celui-ci de façon amovible par l'intermédiaire d'un assemblage par filetage.

2. Système selon la revendication 1, **caractérisé en ce qu'**à partir de la source de lumière (26), qui est réalisée de préférence sous la forme d'une diode laser, un couplage de lumière direct ou un couplage par l'intermédiaire d'un groupe de lentilles (32, 36) est effectué dans l'applicateur.

3. Système selon la revendication 1 ou 2, **caractérisé en ce que** la partie de tête (50) est réalisée pour recevoir les applicateurs et pour un découplage de lumière à travers un alésage central (52), et/ou **en ce qu'**une extrémité avant de la partie de tête (50) s'engage dans le corps de connexion (6) de l'applicateur.

4. Système selon l'une quelconque des revendications 1 à 3, **caractérisé en ce que** l'alésage central (52) est réalisé pour recevoir et/ou centrer l'extrémité de guide de lumière (8) de l'applicateur.

**5.** Système selon l'une quelconque des revendications 1 à 4, **caractérisé en ce qu'**à l'extrémité arrière du tube formant boîtier (42) ou du capuchon (46), un bouton formant clé (48) est prévu au moyen duquel il est possible d'activer ou de désactiver l'appareil d'irradiation.

**6.** Système selon l'une quelconque des revendications 1 à 5, **caractérisé en ce que** les applicateurs sont réalisés sous la forme de systèmes optiques à usage unique pour une seule utilisation.

**7.** Système selon l'une quelconque des revendications 1 à 6, **caractérisé en ce que** le tube formant boîtier (42) présente un témoin de fonctionnement (54).

**8.** Système selon l'une quelconque des revendications 1 à 7, **caractérisé en ce que** dans le tube formant boîtier (42), un film de protection (102) est prévu devant la source de lumière (26) sur la trajectoire du faisceau.

**9.** Système selon l'une quelconque des revendications 1 à 8, **caractérisé en ce que** la partie de tête (50) comprend un mécanisme de verrouillage de telle sorte que la lumière ne peut sortir de l'appareil d'irradiation qu'en cas de connexion complète de l'applicateur avec l'appareil d'irradiation et/ou qu'en cas d'introduction complète du guide de lumière (2) de l'applicateur dans l'appareil d'irradiation.

**10.** Système selon la revendication 9, **caractérisé en ce que** le mécanisme de verrouillage comprend un corps de verrouillage (58) solidaire de l'appareil d'irradiation avec un trou (60) disposé sur la trajectoire du faisceau de la diode laser (26), et **en ce qu'**un disque de verrouillage (62), disposé de façon mobile par rapport au corps de verrouillage (8), est prévu dans un trou (106), dans lequel la trajectoire du faisceau de la source de lumière (26) est coupée au moyen du disque de verrouillage (62) tant que l'applicateur n'est pas connecté à l'appareil d'irradiation, et dans lequel après établissement d'une connexion entre l'applicateur et l'appareil d'irradiation, la trajectoire du faisceau allant de la diode laser (26) dans le guide de lumière (2) de l'applicateur est libérée et/ou le trou prévu dans le disque de verrouillage (62) est aligné sur le trou (60) du corps de verrouillage (58) situé sur la trajectoire du faisceau.

**11.** Système selon l'une quelconque des revendications 1 à 10, **caractérisé en ce que** le corps de connexion (6) de l'applicateur est réalisé sous la forme d'un connecteur à enficher ou à visser muni d'une butée de profondeur.

**12.** Système selon l'une quelconque des revendications 1 à 11, **caractérisé en ce que** le guide de lumière (2) de l'applicateur est guidé à travers le corps de connexion (6) de l'applicateur et dépasse par son extrémité de guide de lumière (8) du corps de connexion (6) vers l'arrière sur une longueur spécifiée, et lorsque la connexion avec l'appareil d'irradiation est établie, s'engage dans la partie de tête (50) de celui-ci.

**13.** Système selon l'une quelconque des revendications 2 à 12, **caractérisé en ce que** la longueur d'onde de la diode laser est de 660 nm.

Fig. 1

## Pocketsonde - senkrecht

### Ausgangsleistung 15,5mW

| Durchmesser | 0,1 mm | 0,2 mm |
|---|---|---|
| Abstand von Lichtquelle zu Glasoberfläche | Gemessene Leistung an Messfläche [mW] | Gemessene Leistung an Messfläche [mW] |
| 1 % Methylenblaulösung | | |
| 0mm | 0,39 | 0 |
| 5mm | 0,38 | 0 |
| 10mm | 0,35 | 0 |
| 15mm | 0,34 | 0 |
| 20mm | 0,33 | 0 |
| 30mm | 0,29 | 0 |
| 0,1% Methylenblaulösung | | |
| 0mm | 8,3 | 3,3 |
| 5mm | 8,1 | 3,3 |
| 10mm | 8 | 3,3 |
| 15mm | 7,7 | 3,3 |
| 20mm | 7,1 | 3,1 |
| 30mm | 5,9 | 2,6 |

Fig. 2

**Fig. 3**

**14**

**Fig. 4**

**Fig. 5**

## Fig. 6

## Fig. 7

**Fig. 8**

**Fig. 9**

## Fig. 10

## Fig. 11

**Fig. 12**

**Fig. 13**

## Fig. 14

**Fig. 15**

## Fig. 16

26

28

32

30

34

36

38

40

## Fig. 17

**Fig. 18**

Applikator A

Applikator B

**Fig. 19**

58

60

64

62

**Fig. 20**

**Fig. 21**

**58**

**62**

**64**

# Fig. 22

## Fig. 23

16,500 -1,0

70
42
44
72
76
78
80
83
90
101
94
100
98
68
102
50

74
82
88
86
56
96
92
97
26

84

6
2

## Fig. 24

## Fig. 25

## Fig. 26

Fig. 27

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10049999 A1
- US 5029581 A
- DE 3918965 A1
- US 5454794 A
- US 4785805 A
- WO 0187416 A1
- EP 0637976 B1